# EUROPEAN PATENT SPECIFICATION

(11) **EP 1 898 935 B1**
(45) Date of publication and mention of the grant of the patent: **19.09.2012**
(21) Application number: 06760547.7
(22) Date of filing: 25.05.2006
(51) Int. Cl.: A61K 31/7072, A61P 31/20

(54) **NORTH-2'-DEOXY-METHANOCARBATHYMIDINES AS ANTIVIRAL AGENTS AGAINST POXVIRUSES**
NORD-2'-DESOXY-METHANOCARBATHYMIDINE ALS ANTIVIRALE MITTEL GEGEN POCKENVIREN
NORD-2'-DESOXY-METHANOCARBATHYMIDINES SERVANT D'AGENTS ANTIVIRAUX CONTRE DES VIRUS POX

(30) Priority: 25.05.2005 US 684811 P
(43) Date of publication of application: 19.03.2008
(73) Proprietor: The Government of the United States of America as represented by The Secretary of the Department of Health and Human Services, Rockville, MD 20852-3804 (US)
(72) Inventor: TSENG, Christopher, K., Burtonsville, MD 20866 (US); MARQUEZ, Victor, E., Montgomery Village, MD 20886 (US)
(74) Representative: Vossius & Partner
(86) International application number: PCT/US2006/020894
(87) International publication number: WO 2006/128159

(56) References cited:
- WO-A-95/08541
- WO-A2-02/08204
- ZALAH LIVNAT ET AL: "Metabolic pathways of N-methanocarbathymidine, a novel antiviral agent, in native and herpes simplex virus type 1 infected Vero cells" ANTIVIRAL RESEARCH, ELSEVIER SCIENCE BV., AMSTERDAM, NL, vol. 55, no. 1, July 2002 (2002-07), pages 63-75, XP002413284 ISSN: 0166-3542
- PRICHARD MARK N ET AL: "Activity and mechanism of action of N-methanocarbathymidine against herpesvirus and orthopoxvirus infections" ANTIMICROBIAL AGENTS AND CHEMOTHERAPY, vol. 50, no. 4, April 2006 (2006-04), pages 1336-1341, XP009079366 ISSN: 0066-4804
- PRICHARD MARK ET AL: "In vitro and in vivo activity of N-methanocarbathymidine against herpesvirus and orthopoxvirus infections and its mechanism of action" ANTIVIRAL RESEARCH, vol. 70, no. 1, May 2006 (2006-05), pages A73-A74, XP009079379 & 19TH INTERNATIONAL CONFERENCE ON ANTIVIRAL RESEARCH; SAN JUAN, PR, USA; MAY 07 -11, 2006 ISSN: 0166-3542
- SMEE DONALD F ET AL: "Cell line dependency for antiviral activity of N-methanocarbathymidine against orthopoxvirus infections" ANTIVIRAL RESEARCH, vol. 70, no. 1, May 2006 (2006-05), page A75, XP009079380 & 19TH INTERNATIONAL CONFERENCE ON ANTIVIRAL RESEARCH; SAN JUAN, PR, USA; MAY 07 -11, 2006 ISSN: 0166-3542

## Description

### FIELD OF THE INVENTION

A pharmaceutical composition comprising an effective amount of an antiviral agent comprising cyclopropanated carbocyclic 2'-deoxynucleoside for use in the prevention or treatment of poxvirus infection in an individual in need thereof is provided.

### BACKGROUND OF THE INVENTION

Poxviruses (members of the Poxviridae family) are the largest and most complex viruses. They are linear double-stranded DNA viruses of 130-300 kilobase pair. The 200-400 nm virion is oval or brick-shaped and can be visualized by light microscopy. The extracellular virion possesses 2 envelopes, while the intracellular virus has only 1 envelope. The virion contains a large number of proteins, at least 10 of which possess enzymatic activity needed for genomic replication. Virus replication is equally complex. Infection is initiated by attachment of the virus to one of several cellular receptors. The virus then can enter the cell by a number of mechanisms. Unlike other DNA viruses, poxviruses replicate in the cytoplasm. The virus contains all the elements for genomic replication, but cellular functions appear necessary for complete viral maturation.

Infections due to the poxviruses occur in humans and animals. The orthopoxviruses include smallpox (variola), monkeypox, vaccinia, and cowpox viruses. Parapoxviruses include orf virus, bovine papular stomatitis virus, pseudocowpox virus, and sealpox virus. Yatapoxviruses include tanapox virus and yabapoxviruses, which are found primarily in Africa. Molluscipoxviruses include the human poxvirus, molluscum contagiosum virus. Smallpox and molluscum are specific to humans. The other viruses cause rare zoonotic infections in humans.

Vaccinia virus can infect humans, and has been used for immunization against smallpox. Its origins are not known but it is believed to be a genetically-distinct type of pox virus which grows readily in a variety of hosts. In man, it causes a localized pustule with scar formation. In immunocompromised persons or eczematous persons, it sometimes causes a severe generalized vaccinia infection. Cowpox is acquired by humans usually by milking cows. It then manifests as ulcerative lesions (sometimes called "milkers nodules") on the hands of dairy workers. Despite its name, rodents are the main reservoir of cowpox; it spreads secondarily to cows and domestic cats. Vaccinia and cowpox are two of only a few experimental animal infections available for evaluation of antiviral compounds against orthopoxviruses.

Smallpox is transmitted by respiratory route from lesions in the respiratory tract of patients in the early stage of the disease. During the 12 day incubation period, the virus is distributed initially to the internal organs and then to the skin. Variola major caused severe infections with 20-50% mortality, variola minor with <1% mortality. Management of outbreaks depended on the isolation of infected individuals and the vaccination of close contacts. The vaccine was highly effective. If given during the incubation period, it either prevented or reduced the severity of clinical symptoms. The origin of the vaccine strain is not known, it is thought that it may have been horsepox which is now an extinct disease. On 8 May 1980, the Thirty-third World Health Assembly declared that smallpox had been eradicated globally.

The discontinuation of routine vaccination has rendered civilian and military populations more susceptible to a disease that is not only infectious by aerosol, but also infamous for its devastating morbidity and mortality. Since 1983, there have existed two WHO-approved and inspected repositories of variola virus: the CDC in the United States and Vector Laboratories in Russia. Despite the promise of variola virus' extinction as a biological entity, the prospect of surreptitious weaponization of smallpox remains vexing. Smallpox virus, used as a weapon against an unimmunized population, has the potential to infect tens of thousands of individuals, kill 30% or more of those infected, and trigger the vaccination of many times that number of individuals.

In most individuals, the smallpox vaccine is safe and effective. However, certain individuals can experience potentially fatal effects, including eczema vaccinatum, progressive vaccinia, and postvaccinal encephalitis. In view of the potential adverse effects associated with the smallpox vaccine, widespread vaccination of the population may not be an acceptable response to the threat of smallpox bioterrorism.

Most studies have focused so far on the efficacy of cidofovir (CDV) against poxviruses. (See De Clercq, et al., Reviews in Medical Virology, September 2004, vol. 14, no. 5, pp. 289-300(12)). This compound has demonstrable activity against cowpox in mice and against monkeypox in monkeys. In the United States, cidofovir may be used in emergencies as an investigational new drug to treat significant adverse events following immunization with the current smallpox vaccine, and in the unlikely event of smallpox reemerging. At present, cidofovir is the drug of choice for therapy of potential smallpox outbreaks and vaccination complications, and an investigational new drug protocol was approved recently for use in response to an actual smallpox outbreak. Although cidofovir is very active against all the orthopoxviruses, it has major limitations in its usefulness. Cidofovir is toxic to kidney tubules and is not active orally, which necessitates intravenous administration. From a practical standpoint, it is anticipated that dosing will be limited to a single dose or to no more than two doses in a smallpox outbreak except for immunocompromised individuals who may require lengthy antiviral therapy. (See Quenelle, et al. Antimicrobial Agents and Chemotherapy, October 2003, p. 3275-3280, Vol. 47, No. 10).

Nucleoside analogs lacking 2'- and 3'-hydroxyl groups (dideoxynucleosides), as well as those 2'-deoxynucleosides where the 3'-hydroxyl function has been chemically modified or changed, can function as chain terminators of DNA synthesis after their triphosphate metabolites are incorporated into DNA. This is the basis of the Sanger dideoxynucleotide method for DNA sequencing (Sanger et al., Proc. Natl. Acad. Sci. USA, 1977). Intense effort has focused on the design and use of these compounds as inhibitors of viral replication (Van Roey et al., Ann. N.Y Acad. Sci., 616:29, 1990). Although the conformation of the sugar moiety in these analogs is believed to play a critical role in modulating biological activity, including the anti-HIV activity mediated by derivatives such as azidothymidine (AZT) and dideoxyinosine (ddI), the main problem encountered in correlating a specific type of sugar conformation with the biological activity of nucleoside analogs is that the sugar ring is quite flexible and its conformation in solution can differ markedly from its conformation in the solid state (Jagannadh et al., Biochem. Biophys. Res. Commun., 179:386; Plavec et al., Biochem. Biophys. Methods, 25:253). Thus, for nucleosides in general, any structure-activity analysis which is based solely on the solid-state conformation would be inaccurate unless it was previously determined that both solution and solid-state conformations were the same.

In solution there is a dynamic equilibrium between Northern (N) and Southern (S) type furanose conformers (Taylor et al., Antiviral Chem. Chemother., 1:163-173, 1990) as defined in the pseudorotational cycle. In this cycle, an absolute Northern conformation corresponds to a range of P (angle of pseudorotation) of from 342° to 18° (₂E → ³T₂ → ³E), whereas an absolute Southern conformation corresponds to a range of P of from 162° to 198° (²E → ²T₃ → ₃E). Preference for any of these specific conformations in solution is determined by the interplay of interactions resulting from anomeric and gauche effects (Saenger, in Principles of Nucleic Acid Structure, Springer-Verlag, New York, pp. 51-104, 1984; Plavec et al., J Am. Chem. Soc., 94:8205-8212, 1972). When a nucleoside or nucleotide binds to its target enzyme, only one form is expected to be present at the active site. While the energy gap between Northern and Southern conformations is about 4 kcal/mol, such a disparity can explain the difference between micromolar and nanomolar binding affinities.

The conformations of nucleosides and their analogs can be described by the geometry of the glycosyl link (syn or anti), the rotation about the exocyclic C4'-C5' bond and the puckering of the sugar ring leading to formation of the twist and envelope conformations. Two types of sugar puckering are generally energetically preferred, namely the C2'-exo/C3'-endo (N or Northern) and the C2'-endo/C3'-exo (S or Southern). The terms "endo" and "exo" refer to displacement of the atom above or below the plane of the ribose ring, respectively. The torsion angles χ [C2-N1-C1'-O4' (pyrimidines) or C4-N9-C1'-O4' (purines)] and y (C3'-C4'-C5'-O5') describe, respectively, the orientations of the base and the 5'-hydroxyl group relative to the ribose ring.

In DNA duplexes, a Southern conformation of the repeating nucleoside unit confers upon the double helix a B-conformation, whereas the Northern conformation induces an A-conformation double helix. The A and B forms of DNA differ in the number of base pairs per turn, the amount of rotation per base pair, the vertical rise per base pair and the helical diameter. In addition, in stretches of DNA containing alternating purines and pyrimidines, a left-handed helix called Z-DNA may form.

Altmann *et al.* demonstrated that substitution of N-methanocarba-thymidine ((N)-methanocarba-T) for thymidine in DNA/RNA heteroduplexes increased the thermodynamic stability of the double helix, as indicated by a positive increase in the Tₘ, whereas the Southern conformer induced a small destabilizing effect (Altmann et al., Tetrahedron Lett., 35:7625-7628, 1994). The increased thermal stability reported for two different (N)-methanocarba-T-containing oligodeoxynucleotides (ODNs) versus conventional ODNs was between 0.8 and 2.1° ° for a single modified nucleotide; however, no data was reported for an ODN containing multiple (N)-methanocarba-Ts in this study. To further elucidate the stabilizing effect of multiple (N)-methanocarba-Ts in the context of the DNA/RNA heteroduplex, a test sequence targeted to the coding region of the SV40 large T-antigen (Wagner, R. W. et al. 1993, Science 260:1510-13) was subsequently synthesized as the phosphorothioate 5'-CTTCATTTTTTCTTC-3' (**SEQ ID NO: 1**), where all thymidines (T) were replaced by (N)-methanocarba-Ts (Marquez, et al., 1996. J. Med. Chem. 39:3739-47). The additive increase in thermodynamic stability of the heteroduplex due to the presence of multiple (N)-methanocarba-T nucleotides was clearly demonstrated with the average stabilization per substitution of ca. 1.3 °C relative to thymidine (Marquez, et al., 1996. J. Med. Chem. 39:3739-47).

Conformationally (Northern) locked nucleoside analogs are described in U.S. Pat. No. 5,629,454 and in U.S. Pat. No. 5,869,666.
WO 02/08204 A2 discloses 5-substituted pyrimidine derivates of conformationally locked nucleoside analogues and that were, *inter alia,* tested against vaccinia and poxvirus.
WO 95/08541 and Zalah et al., Antiviral Research, 55:63-75 (2002), disclose conformationally locked nucleosides, including North-methanocarbathymide (N-MCT).
WO 95/08541 describes an anti-HIV activity and Zalah *et al.,* describes an anti-herpetic activity for N-MCT.

### SUMMARY OF THE INVENTION

The threat from a smallpox bioterrorist attack is a serious health problem for the entire nation. Although early vaccination is a possible recourse, there is no drug available when that window of opportunity is missed, especially if an intentional spread of smallpox occurs in a bioterrorist attack. Accordingly, there is a need for antiviral agents effective against poxviruses, such as smallpox. The compositions of the preferred embodiments provide such agents for use in the treatment.

North-methanocarbathymidine (N-MCT), a thymidine analog with a pseudosugar moiety locked in the northern conformation, which was previously shown to exert strong antiviral activity against herpes simplex virus types 1 and 2 (Marquez, et al., 1996. J. Med. Chem. 39:3739-47), has been identified as exhibiting potent activity against poxviruses, which are DNA viruses. N-MCT effectively blocks poxvirus synthesis through its triphosphate (TP) metabolite, which is more efficiently produced in poxvirus-infected cells. N-MCT is approximately seven times more potent than cidofovir against vaccinia and cowpox. The higher potency and target specificity of N-MCT against poxvirus, as well as its high margin of safety, makes it a highly desirable antiviral agent. In addition, the antiviral mechanism of N-MCT may be different from that of cidofovir, making it even more desirable due to the scarcity of the potential available efficacious anti-pox agents currently under development.
Accordingly, in a first aspect, a compound having the formula: 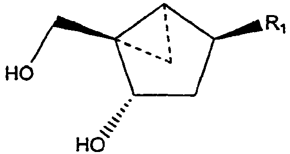 wherein R₁ is a substituted or unsubstituted thymine moiety for use in treating a poxvirus infection in a mammal in need thereof is provided.
In a second aspect, a compound having the formula: 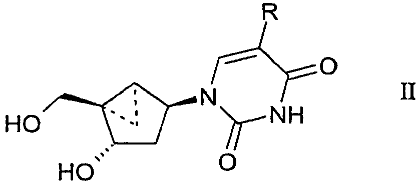 wherein R is

- CH₂-R²;

and
R² is defined as being H,
for use in treating a poxvirus infection in a mammal in need thereof is provided. ,

In an embodiment of the second aspect, the mammal is a human.

In an embodiment of the second aspect, the compound is of the formula:

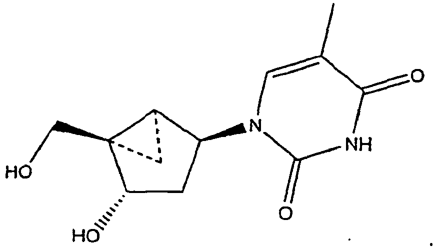

In an embodiment of the second aspect, the poxvirus is smallpox virus.

In an embodiment of the second aspect, the poxvirus is selected from the group consisting of vaccinia, monkeypox, cowpox, rabbitpox, raccoon pox, tatera pox, buffalopox, and camelpox.

In an embodiment of the second aspect, the poxvirus is selected from the group consisting of fowl pox, canary pox, goat pox, sheep pox, lumpy skin disease, myxoma, hare fibroma, orf, pseudo-cowpox, swinepox, molluscum contagiosum, tanapox, yaba, and mousepox.

In an embodiment of the second aspect, the effective antiviral amount is from about 300 mg per day to about 15,000 mg per day.

In an embodiment of the second aspect, the step of administering is selected from the group consisting of topically administering, orally administering, intravenously administering, intramuscularly administering, parenterally administering, intradermally administering, intraperitoneally administering, and subcutaneously administering

In a third aspect, North-methanocarbathymidine triphosphate for use in treating a poxvirus infection in a mammal in need thereof is provided.

In an embodiment of the third aspect, the mammal is a human.

In an embodiment of the third aspect, the poxvirus is smallpox virus.

In an embodiment of the third aspect, the poxvirus is selected from the group consisting of vaccinia, monkeypox, cowpox, rabbitpox, raccoon pox, tatera pox, buffalopox, and camelpox.

In an embodiment of the third aspect, the poxvirus is selected from the group consisting of fowl pox, canary pox, goat pox, sheep pox, lumpy skin disease, myxoma, hare fibroma, orf, pseudo-cowpox, swinepox, molluscum contagiosum, tanapox, yaba, and mousepox.

In a fourth aspect, a pharmaceutical composition comprising an effective amount of a compound having the formula: 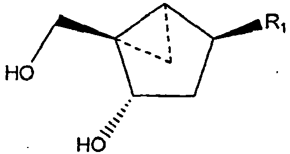 wherein R₁ is a substituted or unsubstituted thymine moiety in a pharmaceutically acceptable carrier for use in the treatment of a poxvirus infection in a patient in need thereof is provided.

In an embodiment of the fourth aspect, the antiviral agent is of the formula: 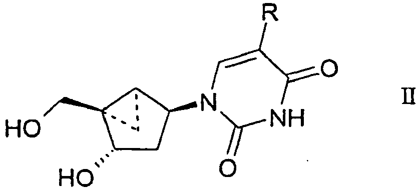 wherein R ist

-CH₂-R²;

and
R² is defined as being H

In an embodiment of the fourth aspect, the antiviral agent is of the formula:

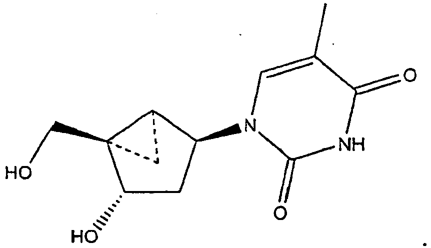

In an embodiment of the fourth aspect, the patient is a human.

In an embodiment of the fourth aspect, the poxvirus is smallpox virus.

In an embodiment of the fourth aspect, the poxvirus is selected from the group consisting of vaccinia, monkeypox, cowpox, rabbitpox, raccoon pox, tatera pox, buffalopox, and camelpox.

In an embodiment of the fourth aspect, the poxvirus is selected from the group consisting of fowl pox, canary pox, goat pox, sheep pox, lumpy skin disease, myxoma, hare fibroma, orf, pseudo-cowpox, swinepox, molluscum contagiosum, tanapox, yaba, and mousepox.

### BRIEF DESCRIPTION OF THE DRAWINGS

Figure 1 depicts multiple amino acid alignments of thymidine kinase homologs expressed in herpes simplex virus 1 (HSV-1), cowpox virus (CV), vaccinia virus (VV) and humans, including a consensus line. Identity and similarity are depicted as light gray and dark gray boxes, respectively.
Figure 2 depicts an unrooted phylogenetic tree based on the amino acid sequence of the thymidine kinase (TK) homologs is shown. Type II orthopoxvirus TK homologs are closely related to the human enzyme that is a member of this same class.
Figure 3 depicts inhibition of VV DNA synthesis by N-MCT. Monolayers of HFF cells were infected with the WR strain of VV and incubated with increasing concentrations of the drugs as illustrated in the figure (0.03, 0.1, 3, 10, and 30 µg/ml). Purified DNA was cut with EcoRV, and a 3,259-bp fragment of VV DNA was detected with a digoxigeninlabeled DNA probe. Numbers at left are molecular sizes in base pairs. CC and VC designate cell control and virus control, respectively.

### DETAILED DESCRIPTION OF THE PREFERRED EMBODIMENT

The carbocyclic 2'-deoxynucleoside analogs of preferred embodiments can be employed in the treatment and prevention of infection by poxviruses. The poxvirus family is subdivided into the entomopoxvirus (EnPV) and chordopoxvirus (ChPV) subfamilies (*Entomopoxvirinae* and *Chordopoxvirinae*), which infect insects and chordates, respectively. The ChPVs are further divided into eight genera (*Avipoxvirus, Molluscipoxvirus, Orthopoxvirus, Capripoxvirus, Suipoxvirus, Leporipoxvirus, Yatapoxvirus* and *Parapoxvirus*), whereas the EnPVs are divided into three genera (*A, B* and *C*). The genus *Orthopoxvirus* includes smallpox (variola), vaccinia, monkeypox, cowpox, rabbitpox, raccoon pox, tatera pox, buffalopox, and camelpox. The genus *Avipoxvirus* includes fowlpox and canary pox. The genus *Capripoxvirus* includes goat pox, sheep pox, and lumpy skin disease. The genus *Leporipoxvirus* includes myxoma and hare fibroma. The genus *Parapoxvirus* includes orf and pseudo-cowpox. The genus *Suipoxvirus* includes swinepox. The genus *Molluscipoxvirus* includes molluscum contagiosum. The genus *Yatapoxvirus* includes tanapox and yaba. Other members of the Poxviridae family include Ectromelia (mousepox) virus. While the preferred embodiments are generally described in relation to smallpox, it is understood that the formulations and methods of preferred embodiments are suitable for use in connection with the specific poxviruses referred to herein as well as other poxviruses. Natural poxviruses, as well as genetically engineered or modified poxviruses and poxviruses resistant to conventional therapies, are also amenable for treatment or prevention by the formulations and methods of preferred embodiments.

### Cyclopropanated Carbocyclic 2'-Deoxynucleosides

Carbocyclic 2'-deoxynucleoside analogs locked in the Northern conformation are effective agents in the prevention and treatment of poxvirus infections. These compounds are described in U.S. Patent No. 5,629,454 and in U.S. Patent No. 5,869,666. Conformationally rigid (locked) nucleoside analogs are constructed on a bicyclo[3.1.0]hexane template whose value of P (pseudorotational angle) fits within the range of absolute Northern or Southern conformations. This bicyclo[3.1.0]hexane template exists exclusively as a pseudoboat, and carbocyclic nucleosides built thereon can adopt either a Northern or Southern conformation, depending on the relative disposition of substituents on the ring. Thus, a Northern C2'-exo (2E) envelope conformation is obtained when the cyclopropane ring was fused between carbon C4' and the carbon supplanting the ribofuranoside oxygen. Conversely, fusion of the cyclopropane ring between carbon C1' and the carbon supplanting the ribofuranoside oxygen provides the opposite Southern conformation. The cyclopropanated carbocyclic 2'-deoxynucleosides of a preferred embodiment as described herein exhibit the following stereochemistry: 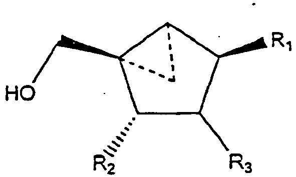 Particularly preferred are compounds of such stereochemistry wherein R₂ is hydroxyl, R₃ is hydrogen, and R₁ is thymine.

While this particular stereochemistry is generally preferred in many embodiments, other stereochemistries can also be preferred in certain other embodiments.

The thymine, cyclopropanated carbocyclic 2'-deoxynucleoside is a particularly preferred compound. This compound has the structure: 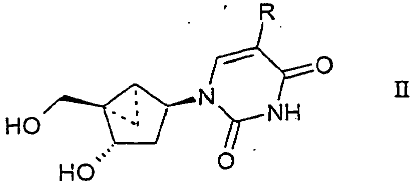 wherein R is

- CH₂-R²;

and
R² is defined as being H

Thymine cyclopropanated carbocyclic 2'-deoxynucleoside is particularly preferred:

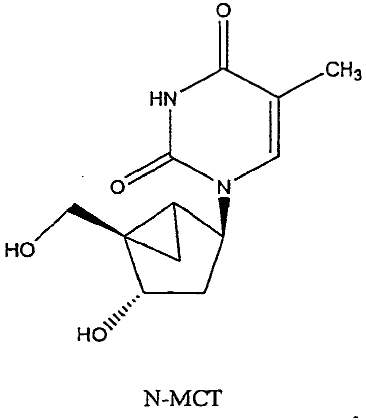

The term "substituted" as used herein is a broad term and is used in its ordinary sense, including, without limitation, to refer to any of the above groups wherein at least one hydrogen atom is replaced with a substituent. In the case of a keto substituent (*i.e.*, -C(=O)-) two hydrogen atoms are replaced. When substituted, "substituents," within the context of preferred embodiments, include halogen, hydroxy, cyano, nitro, amino, alkylamino, dialkylamino, alkyl, alkoxy, alkylthio, haloalkyl, aryl, substituted aryl, arylalkyl, substituted arylalkyl, heteroaryl, substituted heteroaryl, heteroarylalkyl, substituted heteroarylalkyl, heterocycle, substituted heterocycle, heterocyclealkyl, substituted heterocyclealkyl, -NRₐR_{b}, -NRₐC(=O)R_{b}, -NRₐC(=O)NR_{b}R_{c}, -NRₐC(=O)OR_{b}, NRₐSO₂R_{b}, -ORₐ, -C(=O)Rₐ, - C(+O)ORₐ, -C(=O)NRₐR_{b}, -SH, -SRₐ, -SORₐ, -S(=O)₂Rₐ, -OS(=O)₂Rₐ, -OC(=O)NRₐR_{b}, - S(=O)₂ORₐ, wherein Rₐ, R_{b}, and R_{c} are the same or different and are independently selected from hydrogen, alkyl, haloalkyl, substituted alkyl, aryl, substituted aryl, arylalkyl, substituted arylalkyl, heteroaryl, substituted heteroaryl, heteroarylalkyl, substituted heteroarylalkyl, heterocycle, substituted heterocycle, heterocyclealkyl or substituted heterocyclealkyl. When a ring system (*e.g*., a fused ring system, a heterocyclic ring system, a homocyclic ring system, and/or any other ring system) is substituted, the substituents can occupy any location on the ring or a chain attached to the ring.

The compounds of preferred embodiments can incorporate substituents comprising various ring systems. For each ring system, any ring atom capable of being an attachment point can comprise a "point of attachment" of the ring system to the rest of the molecule. Accordingly, in heterocyclic ring systems, any hetero-ring atom can be the point of attachment or occupy another location relative to the point of attachment. In fused ring systems, there can be multiple points of attachments. For rings containing more than one hetero-ring atom, the ring name is intended to cover multiple locations for the hetero-atoms in the ring. For example, the term "triazolyl" encompasses both 1,2,3-triazolyl and 1,2,4-triazole; the term "diazolyl" encompasses 1,2-diazolyl (*i.e.*, pyrazolyl) and 1,3-diazolyl (*i.e*., imidazolyl). Other ring systems wherein the ring name encompasses multiple locations for the heteroatom(s) in the ring include dithiolyl, oxathiolyl, oxazolyl, thiazolyl, oxadiazolyl, oxatriazolyl, dioxazolyl, oxathiazolyl, dioxinyl, triazinyl, oxazinyl, oxathiazinyl, and oxadiazinyl ring systems. Moreover, for fused ring systems wherein at least one ring comprises at least one heteroatom, the heteroatom can occupy any location on the respective ring. For example the term "benzopyridinyl" encompasses benzo[b]pyridinyl (*i.e*., quinolinyl) as well as benzo[c]pyridinyl (*i.e.*, isoquinolinyl). Other fused ring systems wherein the ring name encompasses multiple locations for the heteroatom(s) in the ring include benzofuranyl, benzothiofuranyl, indolyl, pyranopyrrolyl, benzodiazolyl, benzoxazolyl, benzopyranyl, pyridopyridinyl, and benzoxazinyl fused ring systems.

Preferred cyclopropanated carbocyclic 2'-deoxynucleosides include (N)-methanocarba-T (thymidine analog).

This particular cyclopropanated carbocyclic 2'-deoxynucleoside, is depicted by the following structure: 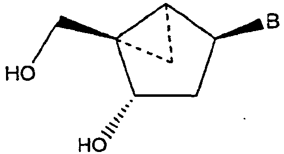 wherein B is thymine

The thymine analogs, especially North-methanocarbathymidine (N-MCT), are particularly preferred because of their exceptionally potent activity against poxviruses such as cowpox and vaccinia.

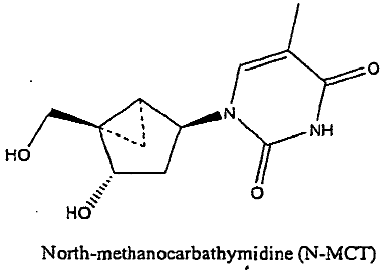

The synthesis of the (N)-methanocarbathymidine (or its adenosine, guanine, cytidine, or uridine analogs) is described below and in Schemes 1-4. The anti-pox effect of various substituted derivatives of the (N)-methanocarba 2'-deoxynucleoside analogs described below can easily be determined by one of ordinary skill in the art using the assay methods described herein without undue experimentation.

Schemes 1-2 can be utilized for the synthesis of intermediate 12, which is chiral, so there is no need for optical resolution at the end of the synthesis, and which can be employed as a starting material for the synthesis of related carbocyclic 2'-deoxynucleoside analogs. Cyclopentenol 6 can be obtained from the sodium borohydride reduction of cyclopentenone 5 (Marquez et al., J. Org. Chem., 53:5709, 1988). Regioselective cleavage of the contiguous O-isopropylidenetriol system in 6 with trimethylaluminum (Takano et al., Tetrahedron Lett., 29:1823, 1988) produces the corresponding carbocyclic 3-tert-butoxy-1,5-glycol 7, which in the presence of tert-butyldimethylsilyl chloride reacts exclusively at the less hindered allylic alcohol position to give the protected intermediate 8. Barton's radical deoxygenation of 8 at C-5 occurs via the xanthate 9 in the presence of AIBN to give compound 10. Deprotection of the silyl ether in 10 by fluoride ion unmasks the hydroxyl group (compound 11) which directs the ensuing cyclopropanation to give compound 12.

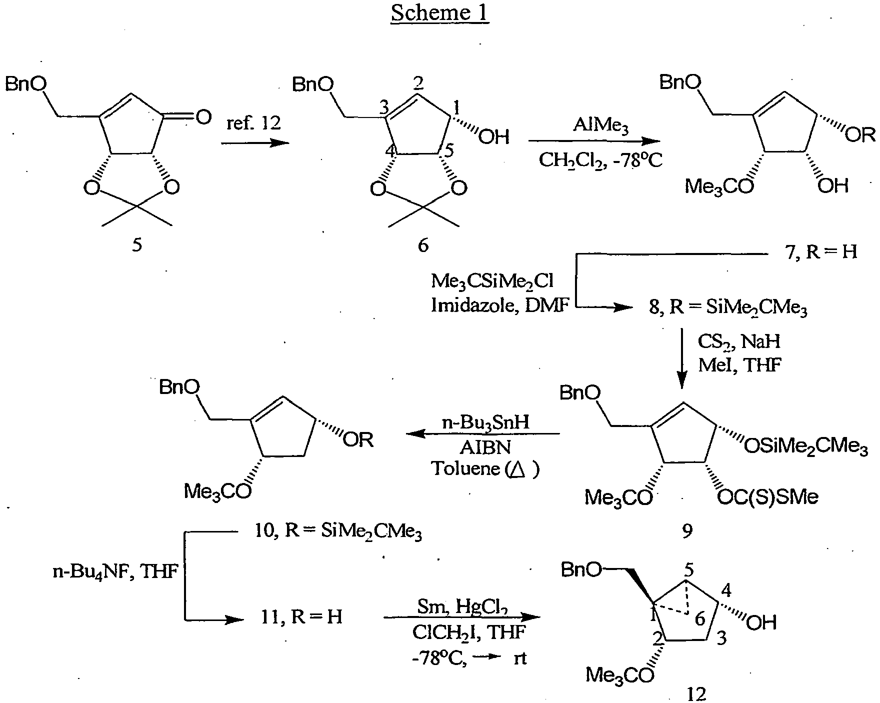

This compound is directly coupled to 6-chloropurine under Mitsunobu conditions (Mitsunobu, Synthesis, 1:1-28, 1981) to give the protected carbocyclic nucleoside intermediate 13. Following aminolysis of 13 with ammonia, and the simultaneous removal of both benzyl and tert-butyl groups, the (N)-2'-deoxy-methanocarba adenosine derivative 4 is obtained.

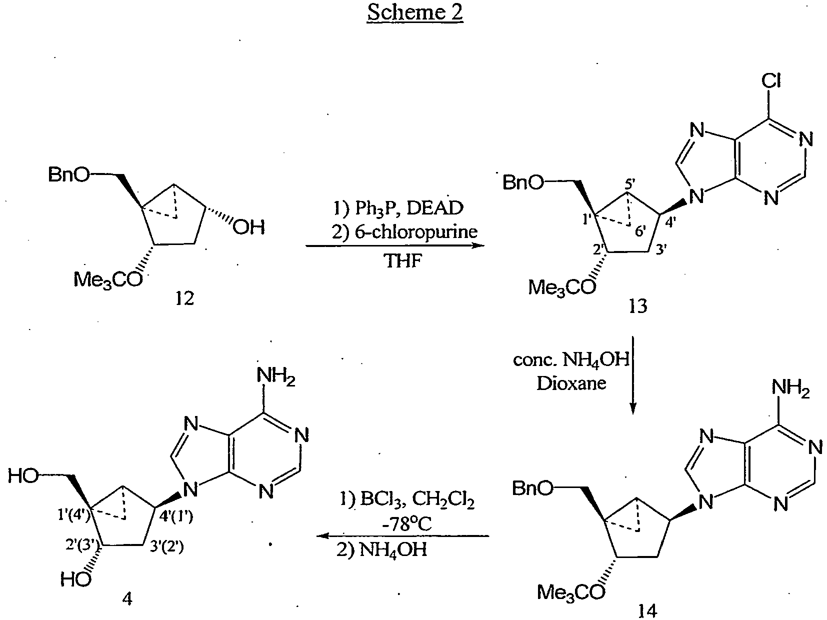

For the pyrimidine derivatives (Scheme 3), protected N³-benzoylthymine and N³-benzoyluracil (Cruickshank et al., Tetrahedron Lett., 25:681, 1994) are coupled according to Scheme 3. In the case of 16, the O-alkylated product predominates, whereas for the uracil analog 17, the situation is reversed. Base-catalyzed deprotection of the N-benzoyl group from intermediates 16 and 17 yields the penultimate intermediates 18 and 19, respectively, and simultaneous removal of both O-benzyl and O-tert-butyl groups with BCl₃ provide the desired targets (N)-methanocarba-T 20 and (N)-methanocarba-U 21. (N)-methanocarba-C 22 is prepared from (N)-methanocarba-U 21 via formation of the triazole intermediate (Divakar et al., J Chem. Soc. Perkin. Trans., I, 1171-1176, 1982).

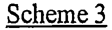

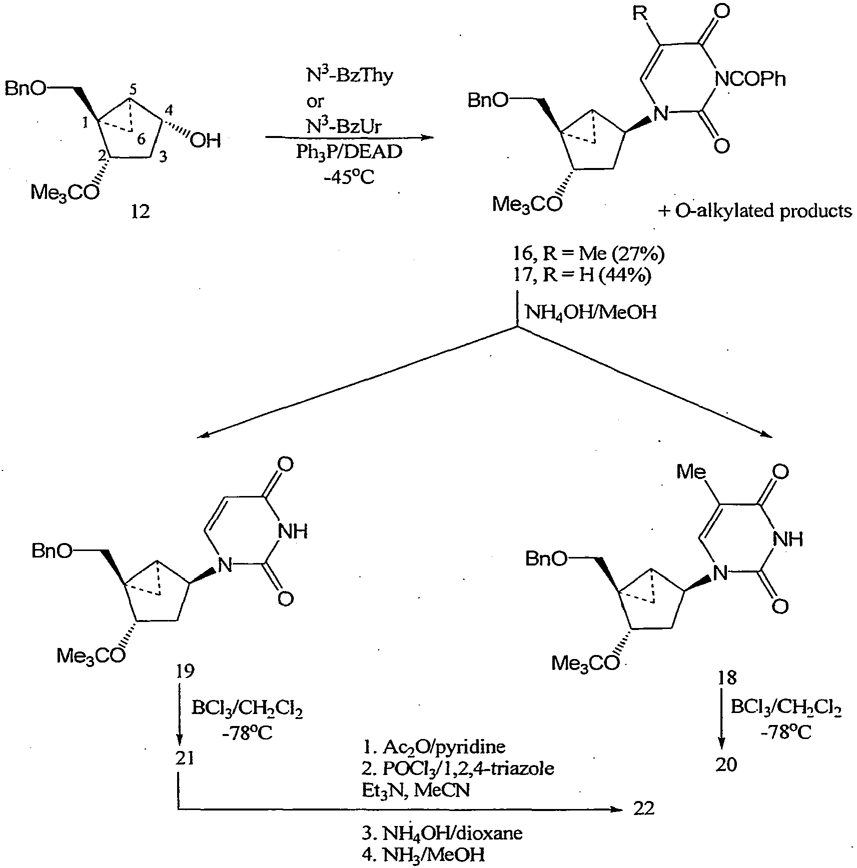

For the synthesis of (N)-methanocarba-G 24 (Scheme 4), coupling under Mitsunobu conditions proceeds with a yield comparable to that of the pyrimidines. Only the desired N-9 isomer (34%) is obtained with virtually no detection of the N-7 isomer. The conversion of the 2-amino-6-chloro intermediate into the 6-0-benzyl derivative 23 facilitates the one-step removal of all protective groups in the generation of the guanine base (Rodriguez et al., Tetrahedron Lett., 34:6233-6236, 1993; Rodriguez et al., J Med. Chem., 37:3389-3399, 1994).

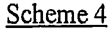

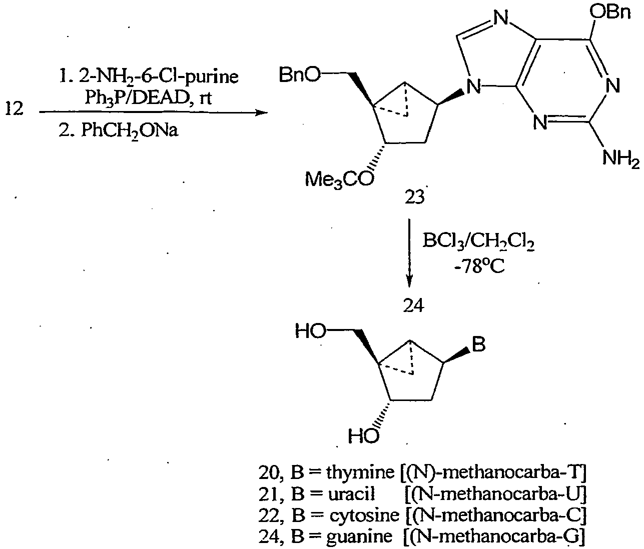

The cyclopropanated carbocyclic 2'-deoxynucleosides of preferred embodiments can also be incorporated into short oligodeoxynucleotides (ODNs). Standard double helices exist in the classic B-DNA form, in which all sugars have a Southern conformation, or in the A-DNA form, wherein the sugars have an N-conformation. During formation of DNA/RNA heteroduplexes, the A-form, typical of RNA, is dominant. The expected thermodynamic stability resulting from the preorganization of the pseudosugar rings into the Northern conformation, typical of A-DNA, is evident by the increase in melting temperature (Tₘ) of the corresponding DNA/RNA heteroduplex containing the (N)-methanocarba T.

### Antiviral Compositions Comprising Cyclopropanated Carbocyclic 2'-Deoxynucleosides

The cyclopropanated carbocyclic 2'-deoxynucleosides (or derivatives, nucleoside prodrugs, or pharmaceutically acceptable esters or salts thereof) of the preferred embodiments, can be incorporated into a pharmaceutically acceptable carrier for administration to an individual having a poxvirus infection, such as a smallpox infection, or can be administered prophylactically to prevent infection upon exposure to a poxvirus. The cyclopropanated carbocyclic 2'-deoxynucleoside can be employed as the sole agent in the prevention or treatment of poxvirus infection, or two or more cyclopropanated carbocyclic 2'-deoxynucleosides can be employed, optionally in combination with other therapeutic agents, *e.g.*, other drugs employed in the treatment of poxvirus infection or in the alleviation of symptoms of poxvirus infection.

The terms "pharmaceutically acceptable salts" and "a pharmaceutically acceptable salt thereof" as used herein are broad terms and are used in their ordinary sense, including, without limitation, to refer to salts prepared from pharmaceutically acceptable, non-toxic acids or bases. Suitable pharmaceutically acceptable salts include metallic salts, *e.g.*, salts of aluminum, zinc, alkali metal salts such as lithium, sodium, and potassium salts, alkaline earth metal salts such as calcium and magnesium salts; organic salts, *e.g.*, salts of lysine, N,N'-dibenzylethylenediamine, chloroprocaine, choline, diethanolamine, ethylenediamine, meglumine (N-methylglucamine), procaine, and tris; salts of free acids and bases; inorganic salts, *e.g.*, sulfate, hydrochloride, and hydrobromide; and other salts which are currently in widespread pharmaceutical use and are listed in sources well known to those of skill in the art, such as, for example, The Merck Index. Any suitable constituent can be selected to make a salt of the cyclopropanated carbocyclic 2'-deoxynucleoside or other therapeutic agents discussed herein, provided that it is non-toxic and does not substantially interfere with the desired activity. In addition to salts, pharmaceutically acceptable precursors and derivatives of the compounds can be employed. Pharmaceutically acceptable amides, lower alkyl esters, and protected derivatives can also be suitable for use in compositions and methods of preferred embodiments.

Contemplated routes of administration include topical, oral, intravenous, subcutaneous, parenteral, intradermal, intramuscular, intraperitoneal, and intravenous, including injectable administration, sustained release from implants, administration by eyedrops, and the like. Nonlimiting examples of particularly preferred nucleoside analog compositions for topical administration include creams, lotions, gels, salves, sprays, dispersions, suspensions, pastes, and ointments.

The cyclopropanated carbocyclic 2'-deoxynucleosides of preferred embodiments can be formulated into liquid preparations for, *e.g.*, oral, nasal, anal, rectal, buccal, vaginal, peroral, intragastric, mucosal, perlingual, alveolar, gingival, olfactory, or respiratory mucosa administration. Suitable forms for such administration include suspensions, syrups, and elixirs. If nasal or respiratory (mucosal) administration is desired (*e.g.*, aerosol inhalation or insufflation), compositions may be in a form and dispensed by a squeeze spray dispenser, pump dispenser or aerosol dispenser. Aerosols are usually under pressure by means of a hydrocarbon. Pump dispensers can preferably dispense a metered dose or a dose having a particular particle size.

The pharmaceutical compositions containing cyclopropanated carbocyclic 2'-deoxynucleosides are preferably isotonic with the blood or other body fluid of the recipient. The isotonicity of the compositions can be attained using sodium tartrate, propylene glycol or other inorganic or organic solutes. Sodium chloride is particularly preferred. Buffering agents can be employed, such as acetic acid and salts, citric acid and salts, boric acid and salts, and phosphoric acid and salts. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like.

Viscosity of the pharmaceutical compositions can be maintained at the selected level using a pharmaceutically acceptable thickening agent. Methylcellulose is preferred because it is readily and economically available and is easy to work with. Other suitable thickening agents include, for example, xanthan gum, carboxymethyl cellulose, hydroxypropyl cellulose, carbomer, and the like. The preferred concentration of the thickener will depend upon the thickening agent selected. An amount is preferably used that will achieve the selected viscosity. Viscous compositions are normally prepared from solutions by the addition of such thickening agents.

A pharmaceutically acceptable preservative can be employed to increase the shelf life of the pharmaceutical compositions. Benzyl alcohol can be suitable, although a variety of preservatives including, for example, parabens, thimerosal, chlorobutanol, or benzalkonium chloride can also be employed. A suitable concentration of the preservative is typically from about 0.02% to about 2% based on the total weight of the composition, although larger or smaller amounts can be desirable depending upon the agent selected.

The cyclopropanated carbocyclic 2'-deoxynucleosides of preferred embodiments can be in admixture with a suitable carrier, diluent, or excipient such as sterile water, physiological saline, glucose, or the like, and can contain auxiliary substances such as wetting or emulsifying agents, pH buffering agents, gelling or viscosity enhancing additives, preservatives, flavoring agents, colors, and the like, depending upon the route of administration and the preparation desired. Standard texts, such as "Remington: The Science and Practice of Pharmacy", Lippincott Williams & Wilkins; 20th edition (June 1, 2003) and "Remington's Pharmaceutical Sciences," Mack Pub. Co.; 18th and 19th editions (December 1985, and June 1990, respectively). Such preparations can include complexing agents, metal ions, polymeric compounds such as polyacetic acid, polyglycolic acid, hydrogels, dextran, and the like, liposomes, microemulsions, micelles, unilamellar or multilamellar vesicles, erythrocyte ghosts or spheroblasts. Suitable lipids for liposomal formulation include, without limitation, monoglycerides, diglycerides, sulfatides, lysolecithin, phospholipids, saponin, bile acids, and the like. The presence of such additional components can influence the physical state, solubility, stability, rate of *in vivo* release, and rate of *in vivo* clearance, and are thus chosen according to the intended application, such that the characteristics of the carrier are tailored to the selected route of administration.

For oral administration, the cyclopropanated carbocyclic 2'-deoxynucleosides can be provided as a tablet, aqueous or oil suspension, dispersible powder or granule, emulsion, hard or soft capsule, syrup or elixir. Compositions intended for oral use can be prepared according to any method known in the art for the manufacture of pharmaceutical compositions and can include one or more of the following agents: sweeteners, flavoring agents, coloring agents and preservatives. Aqueous suspensions can contain the active ingredient in admixture with excipients suitable for the manufacture of aqueous suspensions.

Formulations for oral use can also be provided as hard gelatin capsules, wherein the cyclopropanated carbocyclic 2'-deoxynucleoside is mixed with an inert solid diluent, such as calcium carbonate, calcium phosphate, or kaolin, or as soft gelatin capsules. In soft capsules, the active compounds can be dissolved or suspended in suitable liquids, such as water or an oil medium, such as peanut oil, olive oil, fatty oils, liquid paraffin, or liquid polyethylene glycols. Stabilizers and microspheres formulated for oral administration can also be used. Capsules can include push-fit capsules made of gelatin, as well as soft, sealed capsules made of gelatin and a plasticizer, such as glycerol or sorbitol. The push-fit capsules can contain the cyclopropanated carbocyclic 2'-deoxynucleoside in admixture with fillers such as lactose, binders such as starches, and/or lubricants such as talc or magnesium stearate and, optionally, stabilizers.

Tablets can be uncoated or coated by known methods to delay disintegration and absorption in the gastrointestinal tract and thereby provide a sustained action over a longer period of time. For example, a time delay material such as glyceryl monostearate can be used. When administered in solid form, such as tablet form, the solid form typically comprises from about 0.001 wt. % or less to about 50 wt. % or more of active ingredient(s) including the cyclopropanated carbocyclic 2'-deoxynucleoside, preferably from about 0.005, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1 wt. % to about 2, 3, 4, 5, 6, 7, 8, 9, 10, 15, 20, 25, 30, 35, 40, or 45 wt. %.

Tablets can contain the cyclopropanated carbocyclic 2'-deoxynucleoside in admixture with non-toxic pharmaceutically acceptable excipients including inert materials. For example, a tablet can be prepared by compression or molding, optionally, with one or more additional ingredients. Compressed tablets can be prepared by compressing in a suitable machine the active ingredients in a free-flowing form such as powder or granules, optionally mixed with a binder, lubricant, inert diluent, surface active or dispersing agent. Molded tablets can be made by molding, in a suitable machine, a mixture of the powdered compound moistened with an inert liquid diluent.

Preferably, each tablet or capsule contains from about 10 mg or less to about 1,000 mg or more of the cyclopropanated carbocyclic 2'-deoxynucleoside, more preferably from about 20, 30, 40, 50, 60, 70, 80, 90, or 100 mg to about 150, 200, 250, 300, 350, 400, 450, 500, 550, 600, 650, 700, 750, 800, or 900 mg. Most preferably, tablets or capsules are provided in a range of dosages to permit divided dosages to be administered. A dosage appropriate to the patient and the number of doses to be administered daily can thus be conveniently selected. While it is generally preferred to incorporate the cyclopropanated carbocyclic 2'-deoxynucleoside and any other therapeutic agent employed in combination therewith in a single tablet or other dosage form, *e.g.*, in a combination therapy, in certain embodiments it can be desirable to provide the cyclopropanated carbocyclic 2'-deoxynucleoside and other therapeutic agents in separate dosage forms.

Suitable inert materials include diluents, such as carbohydrates, mannitol, lactose, anhydrous lactose, cellulose, sucrose, modified dextrans, starch, and the like, or inorganic salts such as calcium triphosphate, calcium phosphate, sodium phosphate, calcium carbonate, sodium carbonate, magnesium carbonate, and sodium chloride. Disintegrants or granulating agents can be included in the formulation, for example, starches such as corn starch, alginic acid, sodium starch glycolate, Amberlite, sodium carboxymethylcellulose, ultramylopectin, sodium alginate, gelatin, orange peel, acid carboxymethyl cellulose, natural sponge and bentonite, insoluble cationic exchange resins, powdered gums such as agar, karaya or tragacanth, or alginic acid or salts thereof.

Binders can be used to form a hard tablet. Binders include materials from natural products such as acacia, tragacanth, starch and gelatin, methyl cellulose, ethyl cellulose, carboxymethyl cellulose, polyvinyl pyrrolidone, hydroxypropylmethyl cellulose, and the like.

Lubricants, such as stearic acid or magnesium or calcium salts thereof, polytetrafluoroethylene, liquid paraffin, vegetable oils and waxes, sodium lauryl sulfate, magnesium lauryl sulfate, polyethylene glycol, starch, talc, pyrogenic silica, hydrated silicoaluminate, and the like, can be included in tablet formulations.

Surfactants can also be employed, for example, anionic detergents such as sodium lauryl sulfate, dioctyl sodium sulfosuccinate and dioctyl sodium sulfonate, cationic such as benzalkonium chloride or benzethonium chloride, or nonionic detergents such as polyoxyethylene hydrogenated castor oil, glycerol monostearate, polysorbates, sucrose fatty acid ester, methyl cellulose, or carboxymethyl cellulose.

Controlled release formulations can be employed wherein the cyclopropanated carbocyclic 2'-deoxynucleoside is incorporated into an inert matrix that permits release by either diffusion or leaching mechanisms. Slowly degenerating matrices can also be incorporated into the formulation. Other delivery systems can include timed release, delayed release, or sustained release delivery systems.

Coatings can be used, for example, nonenteric materials such as methyl cellulose, ethyl cellulose, hydroxyethyl cellulose, methylhydroxy-ethyl cellulose, hydroxypropyl cellulose, hydroxypropyl-methyl cellulose, sodium carboxy-methyl cellulose, providone and the polyethylene glycols, or enteric materials such as phthalic acid esters. Dyestuffs or pigments can be added for identification or to characterize different combinations of active compound doses

When administered orally in liquid form, a liquid carrier such as water, petroleum, oils of animal or plant origin such as peanut oil, mineral oil, soybean oil, or sesame oil, or synthetic oils can be added to the cyclopropanated carbocyclic 2'-deoxynucleoside. Physiological saline solution, dextrose, or other saccharide solution, or glycols such as ethylene glycol, propylene glycol, or polyethylene glycol are also suitable liquid carriers. The pharmaceutical compositions can also be in the form of oil-in-water emulsions. The oily phase can be a vegetable oil, such as olive or arachis oil, a mineral oil such as liquid paraffin, or a mixture thereof. Suitable emulsifying agents include naturally-occurring gums such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsions can also contain sweetening and flavoring agents.

Pulmonary delivery of the cyclopropanated carbocyclic 2'-deoxynucleosides of preferred embodiments can also be employed. The cyclopropanated carbocyclic 2'-deoxynucleoside is delivered to the lungs while inhaling and traverses across the lung epithelial lining to the blood stream. A wide range of mechanical devices designed for pulmonary delivery of therapeutic products can be employed, including but not limited to nebulizers, metered dose inhalers, and powder inhalers, all of which are familiar to those skilled in the art. These devices employ formulations suitable for the dispensing of the cyclopropanated carbocyclic 2'-deoxynucleoside. Typically, each formulation is specific to the type of device employed and can involve the use of an appropriate propellant material, in addition to diluents, adjuvants, and/or carriers useful in therapy.

The cyclopropanated carbocyclic 2'-deoxynucleoside and other optional active ingredients are advantageously prepared for pulmonary delivery in particulate form with an average particle size of from 0.1 µm or less to 10 µm or more, more preferably from about 0.2, 0.3, 0.4, 0.5, 0.6, 0.7, 0.8, or 0.9 µm to about 1.0, 1.5, 2.0, 2.5, 3.0, 3.5, 4.0, 4.5, 5.0, 5.5, 6.0, 6.5, 7.0, 7.5, 8.0, 8.5, 9.0, or 9.5 µm. Pharmaceutically acceptable carriers for pulmonary delivery of the cyclopropanated carbocyclic 2'-deoxynucleosides include carbohydrates such as trehalose, mannitol, xylitol, sucrose, lactose, and sorbitol. Other ingredients for use in formulations can include DPPC, DOPE, DSPC, and DOPC. Natural or synthetic surfactants can be used, including polyethylene glycol and dextrans, such as cyclodextran. Bile salts and other related enhancers, as well as cellulose and cellulose derivatives, and amino acids can also be used. Liposomes, microcapsules, microspheres, inclusion complexes, and other types of carriers can also be employed.

Pharmaceutical formulations suitable for use with a nebulizer, either jet or ultrasonic, typically comprise the cyclopropanated carbocyclic 2'-deoxynucleoside dissolved or suspended in water at a concentration of about 0.01 or less to 100 mg or more of cyclopropanated carbocyclic 2'-deoxynucleoside per mL of solution, preferably from about 0.1, 1, 2, 3, 4, 5, 6, 7, 8, 9, or 10 mg to about 15, 20, 25, 30, 35, 40, 45, 50, 55, 60, 65, 70, 75, 80, 85, or 90 mg per mL of solution. The formulation can also include a buffer and a simple sugar (*e.g*., for protein stabilization and regulation of osmotic pressure). The nebulizer formulation can also contain a surfactant, to reduce or prevent surface induced aggregation of the cyclopropanated carbocyclic 2'-deoxynucleoside caused by atomization of the solution in forming the aerosol.

Formulations for use with a metered-dose inhaler device generally comprise a finely divided powder containing the active ingredients suspended in a propellant with the aid of a surfactant. The propellant can include conventional propellants, such as chlorofluorocarbons, hydrochlorofluorocarbons, hydrofluorocarbons, and hydrocarbons. Preferred propellants include trichlorofluoromethane, dichlorodifluoromethane, dichlorotetrafluoroethanol, 1,1,1,2-tetrafluoroethane, and combinations thereof. Suitable surfactants include sorbitan trioleate, soya lecithin, and oleic acid.

Formulations for dispensing from a powder inhaler device typically comprise a finely divided dry powder containing the cyclopropanated carbocyclic 2'-deoxynucleoside, optionally including a bulking agent, such as lactose, sorbitol, sucrose, mannitol, trehalose, or xylitol in an amount that facilitates dispersal of the powder from the device, typically from about 1 wt. % or less to 99 wt. % or more of the formulation, preferably from about 5, 10, 15, 20, 25, 30, 35, 40, 45, or 50 wt. % to about 55, 60, 65, 70, 75, 80, 85, or 90 wt. % of the formulation.

When the cyclopropanated carbocyclic 2'-deoxynucleoside is administered by intravenous, cutaneous, subcutaneous, parenteral, or other injection, it is preferably in the form of a pyrogen-free, parenterally acceptable aqueous solution or oleaginous suspension. Suspensions can be formulated according to methods well known in the art using suitable dispersing or wetting agents and suspending agents. The preparation of acceptable aqueous solutions with suitable pH, isotonicity, stability, and the like, is within the skill in the art. A preferred pharmaceutical composition for injection preferably contains an isotonic vehicle such as 1,3-butanediol, water, isotonic sodium chloride solution, Ringer's solution, dextrose solution, dextrose and sodium chloride solution, lactated Ringer's solution, or other vehicles as are known in the art. In addition, sterile fixed oils can be employed conventionally as a solvent or suspending medium. For this purpose, any bland fixed oil can be employed including synthetic mono or diglycerides. In addition, fatty acids such as oleic acid can likewise be used in the formation of injectable preparations. The pharmaceutical compositions can also contain stabilizers, preservatives, buffers, antioxidants, or other additives known to those of skill in the art.

The duration of the injection can be adjusted depending upon various factors, and can comprise a single injection administered over the course of a few seconds or less, to 1, 2, 3, 4, 5, 6, 7, 8, 9, 10, 11, 12, 13, 14, 15, 16, 17, 18, 19, 20, 21, 22, 23, or 24 hours or more of continuous intravenous administration.

The cyclopropanated carbocyclic 2'-deoxynucleosides can be administered systematically or locally, via a liquid or gel, or as an implant or device.

The anti-pox compositions of the preferred embodiments can additionally employ adjunct components conventionally found in pharmaceutical compositions in their art-established fashion and at their art-established levels. Thus, for example, the compositions can contain additional compatible pharmaceutically active materials for combination therapy (such as supplementary antimicrobials, antipruritics, astringents, local anesthetics, antiinflammatory agents, and the like), or can contain materials useful in physically formulating various dosage forms of the preferred embodiments, such as excipients, dyes, perfumes, thickening agents, stabilizers, skin penetration enhancers, preservatives or antioxidants.

Secondary skin infections are often associated with smallpox infections. Accordingly, it is desirable to administer the cyclopropanated carbocyclic 2'-deoxynucleosides of preferred embodiments in preparations including other therapeutic agents such as anti-microbial agents (anti-bacterials, anti-mycobacterials, anti-virals, anti-fungal, anti-parasites, and the like). Examples of anti-bacterials include beta-lactam antibiotics, penicillins (such as natural penicillins, aminopenicillins, penicillinase-resistant penicillins, carboxy penicillins, ureido penicillins), cephalosporins (first generation, second generation, and third generation cephalosporins), and other beta-lactams (such as imipenem, monobactams), beta-lactamase inhibitors, vancomycin, aminoglycosides and spectinomycin, tetracyclines, chloramphenicol, erythromycin, lincomycin, clindamycin, rifampin, metronidazole, polymyxins, sulfonamides and trimethoprim, and quinolines, Acedapsone; Acetosulfone Sodium; Alamecin; Alexidine; Amdinocillin; Amdinocillin Pivoxil; Amicycline; Amifloxacin; Amifloxacin Mesylate; Amikacin; Amikacin Sulfate; Aminosalicylic acid; Aminosalicylate sodium; Amoxicillin; Amphomycin; Ampicillin; Ampicillin Sodium; Apalcillin Sodium; Apramycin; Aspartocin; Astromicin Sulfate; Avilamycin; Avoparcin; Azithromycin; Azlocillin; Azlocillin Sodium; Bacampicillin Hydrochloride; Bacitracin; Bacitracin Methylene Disalicylate; Bacitracin Zinc; Bambermycins; Benzoylpas Calcium; Berythromycin; Betamicin Sulfate; Biapenem; Biniramycin; Biphenamine Hydrochloride; Bispyrithione Magsulfex; Butikacin; Butirosin Sulfate; Capreomycin Sulfate; Carbadox; Carbenicillin Disodium; Carbenicillin Indanyl Sodium; Carbenicillin Phenyl Sodium; Carbenicillin Potassium; Carumonam Sodium; Cefaclor; Cefadroxil; Cefamandole; Cefamandole Nafate; Cefamandole Sodium; Cefaparole; Cefatrizine; Cefazaflur Sodium; Cefazolin; Cefazolin Sodium; Cefbuperazone; Cefdinir; Cefepime; Cefepime Hydrochloride; Cefetecol; Cefixime; Cefinenoxime Hydrochloride; Cefinetazole; Cefinetazole Sodium; Cefonicid Monosodium; Cefonicid Sodium; Cefoperazone Sodium; Ceforanide; Cefotaxime Sodium; Cefotetan; Cefotetan Disodium; Cefotiam Hydrochloride; Cefoxitin; Cefoxitin Sodium; Cefpimizole; Cefpimizole Sodium; Cefpiramide; Cefpiramide Sodium; Cefpirome Sulfate; Cefpodoxime Proxetil; Cefprozil; Cefroxadine; Cefsulodin Sodium; Ceftazidime; Ceftibuten; Ceftizoxime Sodium; Ceftriaxone Sodium; Cefuroxime; Cefuroxime Axetil; Cefuroxime Pivoxetil; Cefuroxime Sodium; Cephacetrile Sodium; Cephalexin; Cephalexin Hydrochloride; Cephaloglycin; Cephaloridine; Cephalothin Sodium; Cephapirin Sodium; Cephradine; Cetocycline Hydrochloride; Cetophenicol; Chloramphenicol; Chloramphenicol Palmitate; Chloramphenicol Pantothenate Complex; Chloramphenicol Sodium Succinate; Chlorhexidine Phosphanilate; Chloroxylenol; Chlortetracycline Bisulfate; Chlortetracycline Hydrochloride; Cinoxacin; Ciprofloxacin; Ciprofloxacin Hydrochloride; Cirolemycin; Clarithromycin; Clinafloxacin Hydrochloride; Clindamycin; Clindamycin Hydrochloride; Clindamycin Palmitate Hydrochloride; Clindamycin Phosphate; Clofazimine; Cloxacillin Benzathine; Cloxacillin Sodium; Cloxyquin; Colistimethate Sodium; Colistin Sulfate; Coumermycin; Coumermycin Sodium; Cyclacillin; Cycloserine; Dalfopristin; Dapsone; Daptomycin; Demeclocycline; Demeclocycline Hydrochloride; Demecycline; Denofungin; Diaveridine; Dicloxacillin; Dicloxacillin Sodium; Dihydrostreptomycin Sulfate; Dipyrithione; Dirithromycin; Doxycycline; Doxycycline Calcium; Doxycycline Fosfatex; Doxycycline Hyclate; Droxacin Sodium; Enoxacin; Epicillin; Epitetracycline Hydrochloride; Erythromycin; Erythromycin Acistrate; Erythromycin Estolate; Erythromycin Ethylsuccinate; Erythromycin Gluceptate; Erythromycin Lactobionate; Erythromycin Propionate; Erythromycin Stearate; Ethambutol Hydrochloride; Ethionamide; Fleroxacin; Floxacillin; Fludalanine; Flumequine; Fosfomycin; Fosfomycin Tromethamine; Fumoxicillin; Furazolium Chloride; Furazolium. Tartrate; Fusidate Sodium; Fusidic Acid; Gentamicin Sulfate; Gloximonam; Gramicidin; Haloprogin; Hetacillin; Hetacillin Potassium; Hexedine; Ibafloxacin; Imipenem; Isoconazole; Isepamicin; Isoniazid; Josamycin; Kanamycin Sulfate; Kitasamycin; Levofuraltadone; Levopropylcillin Potassium; Lexithromycin; Lincomycin; Lincomycin Hydrochloride; Lomefloxacin; Lomefloxacin Hydrochloride; Lomefloxacin Mesylate; Loracarbef; Mafenide; Meclocycline; Meclocycline Sulfosalicylate; Megalomicin Potassium Phosphate; Mequidox; Meropenem; Methacycline; Methacycline Hydrochloride; Methenamine; Methenamine Hippurate; Methenamine Mandelate; Methicillin Sodium; Metioprim; Metronidazole Hydrochloride; Metronidazole Phosphate; Mezlocillin; Meziocillin Sodium; Minocycline; Minocycline Hydrochloride; Mirincamycin Hydrochloride; Monensin; Monensin Sodium; Nafcillin Sodium; Nalidixate Sodium; Nalidixic Acid; Natamycin; Nebramycin; Neomycin Palmitate; Neomycin Sulfate; Neomycin Undecylenate; Netilmicin Sulfate; Neutramycin; Nifuradene; Nifuraldezone; Nifuratel; Nifuratrone; Nifurdazil; Nifurimide; Nifurpirinol; Nifurquinazol; Nifurthiazole; Nitrocycline; Nitrofurantoin; Nitromide; Norfloxacin; Novobiocin Sodium; Ofloxacin; Ormetoprim; Oxacillin Sodium; Oximonam; Oximonam Sodium; Oxolinic Acid; Oxytetracycline; Oxytetracycline Calcium; Oxytetracycline Hydrochloride; Paldimycin; Parachlorophenol; Paulomycin; Pefloxacin; Pefloxacin Mesylate; Penamecillin; Penicillin G Benzathine; Penicillin G Potassium; Penicillin G Procaine; Penicillin G Sodium; Penicillin V; Penicillin V Benzathine; Penicillin V Hydrabamine; Penicillin V Potassium; Pentizidone Sodium; Phenyl Aminosalicylate; Piperacillin Sodium; Pirbenicillin Sodium; Piridicillin Sodium; Pirlimycin Hydrochloride; Pivampicillin Hydrochloride; Pivampicillin Pamoate; Pivampicillin Probenate; Polymyxin B Sulfate; Porfiromycin; Propikacin; Pyrazinamide; Pyrithione Zinc; Quindecamine Acetate; Quinupristin; Racephenicol; Ramoplanin; Ranimycin; Relomycin; Repromicin; Rifabutin; Rifametane; Rifamexil; Rifamide; Rifampin; Rifapentine; Rifaximin; Rolitetracycline; Rolitetracycline Nitrate; Rosaramicin; Rosaramicin Butyrate; Rosaramicin Propionate; Rosaramicin Sodium Phosphate; Rosaramicin Stearate; Rosoxacin; Roxarsone; Roxithromycin; Sancycline; Sanfetrinem Sodium; Sarmoxicillin; Sarpicillin; Scopafungin; Sisomicin; Sisomicin Sulfate; Sparfloxacin; Spectinomycin Hydrochloride; Spiramycin; Stallimycin Hydrochloride; Steffimycin; Streptomycin Sulfate; Streptonicozid; Sulfabenz; Sulfabenzamide; Sulfacetamide; Sulfacetamide Sodium; Sulfacytine; Sulfadiazine; Sulfadiazine Sodium; Sulfadoxine; Sulfalene; Sulfamerazine; Sulfameter; Sulfamethazine; Sulfamethizole; Sulfamethoxazole; Sulfamonomethoxine; Sulfamoxole; Sulfanilate Zinc; Sulfanitran; Sulfasalazine; Sulfasomizole; Sulfathiazole; Sulfazamet; Sulfisoxazole; Sulfisoxazole Acetyl; Sulfisoxazole Diolamine; Sulfomyxin; Sulopenem; Sultamicillin; Suncillin Sodium; Talampicillin Hydrochloride; Teicoplanin; Temafloxacin Hydrochloride; Temocillin; Tetracycline; Tetracycline Hydrochloride; Tetracycline Phosphate Complex; Tetroxoprim; Thiamphenicol; Thiphencillin Potassium; Ticarcillin Cresyl Sodium; Ticarcillin Disodium; Ticarcillin Monosodium; Ticlatone; Tiodonium Chloride; Tobramycin; Tobramycin Sulfate; Tosufloxacin; Trimethoprim; Trimethoprim Sulfate; Trisulfapyrimidines; Troleandomycin; Trospectomycin Sulfate; Tyrothricin; Vancomycin; Vancomycin Hydrochloride; Virginiamycin; Zorbamycin. Anti-mycobacterials include Myambutol (Ethambutol Hydrochloride), Dapsone (4,4'-diaminodiphenylsulfone), Paser Granules (aminosalicylic acid granules), Priftin (rifapentine), Pyrazinamide, Isoniazid, Rifadin (Rifampin), Rifadin IV, Rifamate (Rifampin and Isoniazid), Rifater (Rifampin, Isoniazid, and Pyrazinamide), Streptomycin Sulfate and Trecator-SC (Ethionamide). Anti-virals include amantidine and rimantadine, ribivarin, acyclovir, vidarabine, trifluorothymidine, ganciclovir, zidovudine, retinovir, interferons, Acemannan; Acyclovir; Acyclovir Sodium; Adefovir; Alovudine; Alvircept Sudotox; Amantadine Hydrochloride; Aranotin; Arildone; Atevirdine Mesylate; Avridine; Cidofovir; Cipamfylline; Cytarabine Hydrochloride; Delavirdine Mesylate; Desciclovir; Didanosine; Disoxaril; Edoxudine; Enviradene; Enviroxime; Famciclovir; Famotine Hydrochloride; Fiacitabine; Fialuridine; Fosarilate; Foscarnet Sodium; Fosfonet Sodium; Ganciclovir; Ganciclovir Sodium; Idoxuridine; Kethoxal; Lamivudine; Lobucavir; Memotine Hydrochloride; Methisazone; Nevirapine; Penciclovir; Pirodavir; Ribavirin; Rimantadine Hydrochloride; Saquinavir Mesylate; Somantadine Hydrochloride; Sorivudine; Statolon; Stavudine; Tilorone Hydrochloride; Trifluridine; Valacyclovir Hydrochloride; Vidarabine; Vidarabine Phosphate; Vidarabine Sodium Phosphate; Viroxime; Zalcitabine; Zidovudine; Zinviroxime and integrase inhibitors. Anti-fungals include imidazoles and triazoles, polyene macrolide antibiotics, griseofulvin, amphotericin B, and flucytosine. Antiparasites include heavy metals, antimalarial quinolines, folate antagonists, nitroimidazoles, benzimidazoles, avermectins, praxiquantel, ornithine decarboxylase inhibitors, phenols (*e.g.*, bithionol, niclosamide); synthetic alkaloid (*e.g*., dehydroemetine); piperazines (*e.g.,* diethylcarbamazine); acetanilide (*e.g*., diloxanide furonate); halogenated quinolines (*e.g.,* iodoquinol (diiodohydroxyquin)); nitrofurans (*e.g*., nifurtimox); diamidines (*e.g.,* pentamidine); tetrahydropyrimidine (*e.g*., pyrantel pamoate); sulfated naphthylamine (*e.g.,* suramin).

Preferred anti-infectives for use in combination with the cyclopropanated carbocyclic 2'-deoxynucleosides of preferred embodiments include Difloxacin Hydrochloride; Lauryl Isoquinolinium Bromide; Moxalactam Disodium; Omidazole; Pentisomicin; Sarafloxacin Hydrochloride; Protease inhibitors of HIV and other retroviruses; Integrase Inhibitors of HIV and other retroviruses; Cefaclor (Ceclor); Acyclovir (Zovirax); Norfloxacin (Noroxin); Cefoxitin (Mefoxin); Cefuroxime axetil (Ceftin); Ciprofloxacin (Cipro); Aminacrine Hydrochloride; Benzethonium Chloride: Bithionolate Sodium; Bromchlorenone; Carbamide Peroxide; Cetalkonium Chloride; Cetylpyridinium Chloride: Chlorhexidine Hydrochloride; Clioquinol; Domiphen Bromide; Fenticlor; Fludazonium Chloride; Fuchsin, Basic; Furazolidone; Gentian Violet; Halquinols; Hexachlorophene: Hydrogen Peroxide; Ichthammol; Imidecyl Iodine; Iodine; Isopropyl Alcohol; Mafenide Acetate; Meralein Sodium; Mercufenol Chloride; Mercury, Ammoniated; Methylbenzethonium Chloride; Nitrofurazone; Nitromersol; Octenidine Hydrochloride; Oxychlorosene; Oxychlorosene Sodium; Parachlorophenol, Camphorated; Potassium Permanganate; Povidone-Iodine; Sepazonium Chloride; Silver Nitrate; Sulfadiazine, Silver; Symclosene; Thimerfonate Sodium; Thimerosal; and Troclosene Potassium.

When the cyclopropanated carbocyclic 2'-deoxynucleoside is employed for the prevention or treatment of poxvirus infection, it is particularly preferred to administer it in combination with other agents employed to treat poxvirus infection, such as cidofovir, and the like. The cyclopropanated carbocyclic 2'-deoxynucleoside can also be administered in conjunction with a smallpox vaccine (*e.g*., the vaccinia vaccine -- a live preparation of the infectious vaccinia virus that does not contain the smallpox virus) in those instances wherein the vaccine is as a precaution against developing the disease after exposure to infection, or in conjunction with Vaccinia Immune Globulin, used to treat complications of the smallpox vaccine.

The cyclopropanated carbocyclic 2'-deoxynucleoside can be provided to an administering physician or other health care professional in the form of a kit. The kit is a package which houses a container which contains the cyclopropanated carbocyclic 2'-deoxynucleoside in suitable form and instructions for administering the pharmaceutical composition to a subject. The kit can optionally also contain one or more additional therapeutic agents. The kit can optionally contain one or more diagnostic tools and instructions for use. For example, a kit containing a composition comprising a cyclopropanated carbocyclic 2'-deoxynucleoside in combination with one or more additional therapeutic agents, such as antiviral, antibacterial, and/or anti-infective agents, can be provided, or separate pharmaceutical compositions containing a cyclopropanated carbocyclic 2'-deoxynucleoside and additional therapeutic agents can be provided. The kit can also contain separate doses of the cyclopropanated carbocyclic 2'-deoxynucleoside for serial or sequential administration. The kit can contain suitable delivery devices, *e.g.*, syringes; inhalation devices, and the like, along with instructions for administrating the cyclopropanated carbocyclic 2'-deoxynucleoside and any other therapeutic agent. The kit can optionally contain instructions for storage, reconstitution (if applicable), and administration of any or all therapeutic agents included. The kits can include a plurality of containers reflecting the number of administrations to be given to a subject. In a particularly preferred embodiment, a kit for the treatment of smallpox infection is provided that includes a cyclopropanated carbocyclic 2'-deoxynucleoside and one or more of smallpox vaccine, Vaccinia Immune Globulin, cidofovir, or one or more antibiotic formulations for treating secondary skin infections. The kit can also include instructions, an assay, or a diagnostic for determining if a patient has been exposed to smallpox virus or has an active smallpox infection, or to diagnose or characterize a secondary skin infection or other condition associated with smallpox infection.

The cyclopropanated carbocyclic 2'-deoxynucleosides of preferred embodiments can be administered prophylactically for the prevention of smallpox infection in exposed individuals. Alternatively, therapy is preferably initiated as early as possible following the onset of signs and symptoms of smallpox infection. The administration route, amount administered, and frequency of administration will vary depending on the age of the patient, condition to be treated, and severity of the condition. Contemplated amounts, dosages, and routes of administration for smallpox infections are similar to those established for the treatment of other viral infections using commercially available antiviral agents. Detailed information relating to administration and dosages of antiviral agents can be found in the Physician's Desk Reference, 47th edition, pp. 844-850, 1993 and in Hayden et al., "Antiviral Agents" in Basic Principles in the Diagnosis of Infectious Diseases, pp. 271-274. This information can be adapted in designing treatment regimes utilizing the cyclopropanated carbocyclic 2'-deoxynucleosides of preferred embodiments.

Contemplated amounts of cyclopropanated carbocyclic 2'-deoxynucleosides for oral administration to treat smallpox infections are from about 10 mg or less to about 2000 mg or more administered from about every 4 hours or less to about every 6 hours or more (or from about 4 times daily to about 6 times daily) for from about 5 days or less to about 10 days or more (40 mg/day or less to about 15,000 mg/day or more) or until there is a significant improvement in the condition. To prevent or inhibit the onset of infection in susceptible or exposed individuals, doses of from about 10 mg or less to about 1000 mg or more are orally administered once, twice, or multiple times a day, typically for up to about 12 months, or, in certain circumstances, indefinitely (from about 10 mg/day to about 1,000 mg/day). When treatment is long term, it can be desirable to vary the dosage, employing a higher dosage early in the treatment, and a lower dosage later in the treatment. For topical administration to skin rash or lesions associated with smallpox, a topical preparation containing from about 10 mg or less to about 100 mg or more cyclopropanated carbocyclic 2'-deoxynucleoside per gram of preparation is typically applied in an amount sufficient to adequately cover all lesions, or lesions on only selected areas of the body. Higher or lower dosages can be desirable, depending upon the patient being treated. The topical preparation is applied every three to six hours from four to six times a day for about 5 days or less to 10 days or more or until the lesions have scabbed over (from about 100 mg/day or less to about 1,000 mg/day or more). The dose size per application can be adjusted depending upon the total affected area, but preferably approximates a one cubic centimeter ribbon of preparation per sixteen square centimeters of skin surface area. For intravenous administration, from about 1 mg/kg to about 10 mg/kg is infused at a constant rate over 30 minutes or less to about 1 hour, 2 hours or more, every 6 hours or less to 8 hours or more (typically, from about 3 mg/kg/day to about 30 mg/kg/day) for about 5 days or less to about 7 days or more.

### Experiment #1

### General Approach for Determining Antiviral Activity and Toxicity

The cyclopropanated carbocyclic 2'-deoxynucleosides of preferred embodiments were tested for toxicity and antiviral activity against orthopoxviruses using the methodology developed by E. Kern, Ph.D., University of Alabama Birmingham (see, *e.g.*, Prichard, et al., Antimicrobial Agents and Chemotherapy, Apr. 2006, p. 1336-1341; Prichard, et al., Antiviral Research, received July 25, 2005, accepted January, 19 2006, article in press). The experimental approach was based upon the following: 1) an inexpensive, rapid assay such as a cytopathic effect inhibition assay that is semi-automated was used initially to screen out the negatives; 2) all screening assays were conducted in low-passaged human cells; 3) each assay system contained cidofovir (CDV) as a positive control and acyclovir (ACV) as a negative control; 4) efficacy was preferably demonstrated by at least two different assay systems that detect functional biologic activity; 5) efficacy against vaccinia virus and cowpox virus was preferably confirmed using other isolates; 6) toxicity was determined using both resting and proliferating human fibroblast cells; and 7) for selected compounds, toxicity in rodent myeloid and erythroid progenitor cells was assessed.

### Screening Assay Systems for Determining Antiviral Activity against Vaccinia Virus and Cowpox Virus

Experimental compounds were initially screened for activity using the CPE assay in HFF cells. Further testing in two other cells lines, Vero and MRC-5, and against other strains of virus was conducted for selected compounds that demonstrated activity in other assay systems. All of the screening assay systems utilized were selected to show specific inhibition of a biologic function, *i.e.*, cytopathic effect in susceptible human cells. In the CPE-inhibition assay, the experimental compound was added 1 hr prior to infection so that the assay system would have maximum sensitivity and detect inhibitors of early replicative steps, such as adsorption or penetration, as well as later events. To rule out non-specific inhibition of virus binding to cells, all experimental compounds that showed reasonable activity in the CPE assay were confirmed using a classical plaque reduction assay in which the experimental compound was added 1 hr after infection. These assay systems also can be manipulated by increasing the pre-treatment time in order to demonstrate antiviral activity with oligodeoxynucleotides and/or peptides. By delaying the time of addition of experimental compound after infection, information regarding which step in the virus life cycle is inhibited (*i.e*., early vs. late functions) can be gained. A direct inactivation assay can also be employed to determine the virucidal activity of selected experimental compounds.

### Efficacy

In all the assays used for primary screening, a minimum of six experimental compound concentrations was used covering a range of 100µg/ml to 0.03µg/ml, in 5-fold increments. These data permitted acceptable dose response curves to be obtained. From these data, the dose that inhibited viral replication by 50% (effective concentration 50; EC₅₀) was calculated using the computer software program MacSynergy II by M.N. Prichard, K. R. Asaltine, and C. Shipman, Jr., University of Michigan, Ann Arbor, Michigan.

### Toxicity

The same experimental compound concentrations used to determine efficacy were also used on uninfected cells in each assay to determine toxicity of each experimental compound. The experimental compound concentration that was cytotoxic to cells as determined by their failure to take up a vital stain, neutral red, (cytotoxic concentration 50; CC₅₀) was determined as described above. The neutral red uptake assay was reliable and reproducible and allowed quantitation of toxicity based on the number of viable cells rather than cellular metabolic activity. A cell proliferation assay using HFF cells is a very sensitive assay for detecting experimental compound toxicity to dividing cells and for determining the experimental compound concentration that inhibits cell growth by 50% (IC₅₀) is calculated as described above. In comparison with four human diploid cell lines and Vero cells, HFF cells are the most sensitive and predictive of toxicity for bone marrow cells.

### Assessment of Drug Activity

To determine if each experimental compound has a sufficient antiviral activity that exceeds its level of toxicity, a selectivity index (SI) was calculated according to CC₅₀/EC₅₀. This index, also referred to as a therapeutic index, was used to determine if a compound warranted further study. For these studies, a compound that had an SI of 10 or greater was evaluated in additional assay systems.

### Confirmation of Antiviral Activity and Toxicity for Vaccinia Virus and Cowpox Virus Assay

Experimental compounds that showed activity in the CPE-inhibition assay were confirmed using the plaque reduction assay. Descriptions of the plaque reduction assay are provided in Keith et al., Antimicrobial Agents and Chemotherapy, July 2003, p. 2193-2198; and Keith et al., Antimicrobial Agents and Chemotherapy, May 2004, p. 1869-1871. Susceptibility of additional virus strains of vaccinia virus and activity in other cell types was also determined for selected experimental compounds.

### Toxicity

In addition to the toxicity component incorporated into each assay system, a standardized cell cytotoxicity assay using a vital stain uptake (Neutral Red) was performed using 7 days of drug exposure to confluent non-dividing cells. A cell proliferation assay using HFF cells is a very sensitive assay for detecting drug toxicity to dividing cells and the drug concentration that inhibits cell growth by 50% (IC₅₀) was calculated as described above.

### Laboratory Procedures for Determining Antiviral Efficacy and Toxicity

### Preparation of Experimental Compounds for In vitro Testing

The experimental compound was weighed using an analytical balance and reconstituted in the appropriate vehicle. If the compound is water soluble, it is dissolved in tissue culture medium without serum at 1 mg/ml and diluted for use as indicated below in the description of the assay system. If the compound is not water soluble, then it is automatically dissolved in DMSO at a concentration of 10 mg/ml and diluted for use in each assay. When DMSO or other solvents are used, control cultures receive media containing the same concentration of solvent as test cultures. Compounds that are not sufficiently soluble are generally not preferred for use as antiviral agents; however, they can be modified chemically to increase their solubility.

### Screening and Confirmation Assays for Vaccinia Virus and Cowpox Virus

### Preparation of Human Foreskin Fibroblast (HFF) Cells

Newborn human foreskins were obtained as soon as possible after circumcision and placed in minimal essential medium (MEM) containing vancomycin, fungizone, penicillin, and gentamicin at the usual concentrations, for 4h. The medium was then removed, the foreskin minced into small pieces and washed repeatedly with phosphate buffered saline (PBS) deficient in calcium and magnesium (PD) until red cells were no longer present. The tissue was then trypsinized using trypsin at 0.25% with continuous stirring for 15 min at 37°C in a CO₂ incubator. At the end of each 15 min period, the tissue was allowed to settle to the bottom of the flask. The supernatant containing cells was poured through sterile cheesecloth into a flask containing MEM and 10% fetal bovine serum. The flask containing the medium was kept on ice throughout the trypsinizing procedure. After each addition of cells, the cheesecloth was washed with a small amount of MEM containing serum. Fresh trypsin was added each time to the foreskin pieces and the procedure repeated until all the tissue was digested. The cell-containing medium was then centrifuged at 1000 RPM at 4°C for 10 min. The supernatant liquid was discarded and the cells resuspended in a small amount of MEM with 10% fetal bovine serum (FBS). The cells were then placed in an appropriate number of 25 cm² tissue culture flasks. As cells became confluent and needed trypsinization, they were expanded into larger flasks. The cells were kept on vancomycin and fungizone to passage four, and maintained on penicillin and gentamicin. Cells were used only through passage 10.

### Cytopathic Effect Inhibition Assay

Low passage HFF cells were seeded into 96 well tissue culture plates 24h prior to use at a cell concentration of 2.5 x 10⁵ cells per ml in 0.1 ml of MEM supplemented with 10% FBS. The cells were then incubated for 24h at 37°C in a CO₂ incubator. After incubation, the medium was removed and 125 µl of experimental drug was added to the first row in triplicate wells, all other wells having 100µl of MEM containing 2% FBS. The experimental compound in the first row of wells was then diluted serially 1:5 throughout the remaining wells by transferring 25µl using the BioMek 2000 Laboratory Automation Workstation. After dilution of the experimental compound, 100 µl of the appropriate virus concentration was added to each well, excluding cell control wells, which received 100 µl of MEM. The virus concentration utilized was 1000 PFUs per well. The plates were then incubated at 37°C in a CO₂ incubator for 7 days. After the incubation period, media was aspirated and the cells stained with a 0.1% crystal violet in 3% formalin solution for 4h. The stain was removed and the plates were rinsed using tap water until all excess stain was removed. The plates were allowed to dry for 24h and then read on a BioTek Multiplate Autoreader at 620 nm. The EC₅₀ values were determined by comparing experimental compound treated and untreated cells using a computer program.

### Plaque Reduction Assay using Semi-Solid Overlay

Two days prior to use, HFF cells were plated into 6 well plates and incubated at 37°C with 5% CO₂ and 90% humidity. On the date of assay, the experimental compound was made up at twice the desired concentration in 2X MEM and then serially diluted 1:5 in 2X MEM using 6 concentrations of experimental compound. The initial starting concentration was usually 200 µg/ml down to 0.06 µg/ml. The virus to be used was diluted in MEM containing 10% FBS to a desired concentration which gave 20-30 plaques per well. The media was then aspirated from the wells and 0.2 ml of virus was added to each well in duplicate with 0.2 ml of media being added to drug toxicity wells. The plates were then incubated for 1 h with shaking every 15 min. After the incubation period, an equal amount of 1% agarose was added to an equal volume of each drug dilution. This gives final experimental compound concentrations beginning with 100µg/ml and ending with 0.03 µg/ml and a final agarose overlay concentration of 0.5%. The drug/agarose mixture was applied to each well in 2 ml volume and the plates were incubated for 3 days, after which the cells were stained with a 0.01% solution of neutral red in phosphate buffered saline. After a 5-6h incubation period, the stain was aspirated, and plaques counted using a stereomicroscope at 10X magnification.

### Screening and confirmation assays for toxicity Neutral red uptake assay

Twenty-four hours prior to assay, HFF cells were plated into 96 well plates at a concentration of 2.5 x 10⁴ cells per well. After 24 h, the media was aspirated and 125 µl of experimental compound was added to the first row of wells and then diluted serially 1:5 using the BioMek 2000 Laboratory Automation Workstation in a manner similar to that used in the CPE assay. After drug addition, the plates were incubated for 7 days in a CO₂ incubator at 37°C. At this time, the media/experimental compound was aspirated and 200 µl/well of 0.01% neutral red in PBS was added. This was incubated in the CO₂ incubator for 1h. The dye was aspirated and the cells were washed using a Nunc Plate Washer. After removing the PBS, 200 µg/well of 50% ETOH/1% glacial acetic acid (in H₂O) was added. The plates were rotated for 15 min and the optical densities read at 540 nm on a plate reader. The EC₅₀ values were determined by comparing experimental compound treated and untreated cells using a computer program.

### Cell proliferation assay

Twenty-four hours prior to assay, HFF cells were seeded in 6-well plates at a concentration of 2.5 x 10⁴ cells per well in MEM containing 10% FBS. On the day of the assay, the experimental compound was diluted serially in MEM containing 10% FBS at increments of 1:5 covering a range from 100 µg/ml to 0.03 µg/ml. For experimental compounds that have to be solubilized in DMSO, control wells receive MEM containing 1% DMSO. The media from the wells was aspirated and 2 ml of each experimental compound was then added to each well. The cells were incubated in a CO₂ incubator at 37°C for 72 h. At the end of this time, the media-drug solution was removed and the cells washed. One ml of 0.25% trypsin was added to each well and incubated until the cells started to come off of the plate. The cell-media mixture was then pipetted up and down vigorously to break up the cell suspension and 0.2 ml of the mixture was added to 9.8 ml of Isoton III and counted using a Coulter Counter. Each sample was counted 3 times with 2 replicate wells per sample.

### Bone marrow clonogenic assays

*In vitro* toxicity to bone marrow progenitor cells can be determined by inhibition of myeloid (colony-forming units granulocyte/macrophage (CFU-GM)) and erythroid (burst-forming unit-erythroid (BFU-E)) colony formation in soft agar clonal assays. Using a 21-23 gauge needle attached to a syringe, rodent bone marrow cells are collected from the leg bone of rats or mice by flushing with Isocoves' Modified Dulbecco's medium (IMDM). A single cell suspension is obtained by repeated aspiration through the needle. Nucleated cells are enumerated with a hemacytometer and adjusted to the desired cell concentration in IMDM. Murine CFU-GM assays are prepared with 2.5 x 10⁵ nucleated cells/ml, 20% FBS, 10 ng/ml rmGM-CSF, and 0.2% agarose. BFU-E cultures include 30% FBS, 1% deionized BSA, 0.1 mM 2-ME, 4 U/ml rhEpo, 10 ng/ml rmIL-3, 2.5 x 10⁵ nucleated cells/ml and 0.2% agarose. Triplicate wells (in 6 well plates) containing 0.1ml of experimental compound (10X) receive 1 ml of either culture mixture for each concentration group and slowly mixed. The cultures are allowed to gel at 4°C and then incubated for 7 (CFU-GM) or 9 (BFU-E) days at 37°C in a humidified atmosphere of 5% CO₂ in air. Colonies are counted using an inverted microscope. CFU-GM colonies are identified as cell clones containing at least 40 cells. BFU-E cultures are stained with dianisidine, and aggregates of greater than 60 hemoglobin-containing cells are counted as erythroid colonies. The median inhibitory concentration (IC₅₀) and the 90% inhibitory concentration (IC₉₀) are derived from linear regression analysis of the logarithm of drug concentration versus CFU-GM or BFU-E survival fraction.

### Plaque Reduction and Cytopathic Effect Inhibition Assay Results

Assays were conducted as described above to directly measure the extent to which N-MCT, N-MCBrU, and CVD (a positive control drug) inhibited the effects of infection by vaccinia virus (Copenhagen Strain) and cowpox virus (Brighton strain) in Human Foreskin Fibroblast (HFF) tissue culture. Results of the testing (Table 1) indicated that N-MCT exhibited superior antiviral potency compared to CDV. N-MCT was more active than M-MCBrU, and that M-MCBrU was more active than CVD. Activity in the plaque assay for N-MCT was approximately 7-fold higher than for CVD. All compounds tested were non-cytotoxic.

**Table 1.**

| | | Vaccinia (Copenhagen) | | | Cowpox (Brighton) | |
|---|---|---|---|---|---|---|
| Drug | Assay | CC₅₀ (µg/ml) | EC₅₀ (µg/ml) | SI | EC₅₀ (µg/ml) | SI |
| N-MCT | CPE | >100 | 0.73 | >137 | 0.31 | >323 |
| N-MCBrU | CPE | >100 | 0.6 | >156 | 11.5 | >8.5 |
| CDV | CPE | >100 | 1.8 | >56 | 2.1 | >48 |
| N-MCT | Plaque reduction | >100 | 0.46 | >217 | 0.6 | >167 |
| N-MCT | Plaque reduction | >100 | 2.6 | >38.5 | 3.4 | >29.4 |
| CDV | Plaque reduction | >100 | 3.5 | >29 | 4.1 | >25 |

| | | | | | | |
|---|---|---|---|---|---|---|
| EC₅₀ (µg/ml) (50% Effective Concentration) = concentration required to inhibit virus-induced effect by 50% CC₅₀ (50% Cytotoxic Concentration) = concentration required to cause cytotoxic effect by 50% SI (Selective Index) = CC₅₀/EC₅₀ | | | | | | |

### BALB/c Mice Inoculated with Vaccinia IHD

The effect of twice daily i.p. treatment with N-MCT (compared to placebo or CDV) on the mortality of BALB/c mice inoculated intranasally with vaccinia IHD strain was investigated. N-MCT was prepared in 0.4 % CMC and delivered i.p. in 0.1 ml doses. CDV was prepared in sterile saline and given i.p. in 0.1 ml doses. Animals were treated twice daily for 5 days beginning 24 hours post viral inoculation. Results of the testing (Table 2) indicated that N-MCT exhibited similar *in vivo* antiviral potency compared to CDV. Because of the reduced toxicity of N-MCT compared to CVD, higher dosages were administered without increased mortality.

**Table 2.**

| Treatment | Mortality | | P-value | Mean Day of Death | P-value |
|---|---|---|---|---|---|
| | Number | Percent | | | |
| Placebo - Saline | 15/15 | 100 | - | 7.6 | - |
| CDV (15 mg/kg) | 0/15 | 0 | 0.001 | - | - |
| N-MCT (50 mg/kg) | 0/15 | 0 | <0.001 | - | - |
| N-MCT(16.7 mg/kg) | 0/15 | 0 | <0.001 | - | - |
| N-MCT(5.6 mg/kg) | 3/15 | 20 | <0.001 | 8.7 | <0.05 |

### BALB/c Mice Inoculated with Cowpox Brighton Strain

The effect of twice daily i.p. treatment with N-MCT (compared to placebo or CDV) on the mortality of BALB/c mice inoculated intranasally with cowpox was investigated. N-MCT was prepared in 0.4 % CMC and delivered i.p. in 0.1 ml doses. CDV was prepared in sterile saline and given i.p. in 0.1 ml doses. Animals were treated twice daily for 5 days beginning 24 hours post viral inoculation. Results of the testing (Table 3) indicated that N-MCT exhibited satisfactory *in vivo* antiviral potency slightly less than that of CDV. Because of the reduced toxicity of N-MCT compared to CVD, higher dosages were administered without increased mortality.

**Table 3.**

| Treatment | Mortality | | P-value | Mean Day of Death | P-value |
|---|---|---|---|---|---|
| | Number | Percent | | | |
| Placebo - Saline | 15/15 | 100 | - | 9.6 | - |
| CDV (15 mg/kg) | 0/15 | 0 | <0.01 | - | - |
| N-MCT (50 mg/kg) | 2/15 | 13 | <0.001 | 7.5 | Not Significant |
| N-MCT(16.7 mg/kg) | 3/15 | 20 | <0.001 | 13.3 | 0.01 |
| N-MCT(5.6 mg/kg) | 6/15 | 40 | <0.001 | 14.0 | 0.05 |

### BALB/c Mice Inoculated with Cowpox Brighton Strain

The effect of twice daily i.p. treatment with N-MCT (compared to placebo or CDV) on the mortality of BALB/c mice challenged with cowpox was investigated. N-MCT was prepared in 0.4 % CMC and delivered i.p. in 0.1 ml doses to treat the resulting infection. CDV was prepared in sterile saline and given i.p. in 0.1 ml doses. Animals were treated twice daily for 7 days beginning 1 day after viral challenge. Results of the testing (Table 4) indicated that N-MCT exhibited similar *in vivo* antiviral potency compared to CDV.

**Table 4.**

| Treatment | Number of Survivors |
|---|---|
| Placebo - Saline | 0/10 |
| CDV (100 mg/kg/day) | 10/10 |
| N-MCT (100 mg/kg/day) | 9/10 |
| N-MCT(30 mg/kg/day) | 2/10 |
| N-MCT(10 mg/kg/day) | 1/10 |

### BALB/c Mice Inoculated with Vaccinia WR Strain

The effect of twice daily i.p. treatment with N-MCT (compared to placebo or CDV) on the mortality of BALB/c mice challenged with vaccinia WR strain, a more virulent strain than the vaccinia IHD strain, was investigated. N-MCT was prepared in 0.4 % CMC and delivered i.p. in 0.1 ml doses to treat the resulting infection. CDV was prepared in sterile saline and given i.p. in 0.1 ml doses. Animals were treated twice daily for 7 days beginning 1 day after viral challenge. Results of the testing (Table 5) indicated that N-MCT did not exhibit efficacy against the vaccinia WR strain.

**Table 5.**

| Treatment | Number of Survivors |
|---|---|
| Placebo - Saline | 0/20 |
| CDV (100 mg/kg/day) | 10/10 |
| N-MCT (100 mg/kg/day) | 0/10 |
| N-MCT(30mg/kg/day) | 0/10 |
| N-MCT(10 mg/kg/day) | 0/10 |

The above described experiments were repeated. The results of the experiments regarding activity of N-MCT against a severe vaccinia (WR strain) respiratory infection in mice (high virus challenge of 10⁵ PFU/mouse) is presented in Table 6.

**Table 6.**

| Compound (mg/kg/day)*^{a}* | Treatment Days | Survivors/Total | MDD*^{b}* | Mean Lung Virus Titer*^{c}* |
|---|---|---|---|---|
| N-MCT(100 mg/kg/day) | 1-7 | 7/10*** | 8.3 ± 0.6 | 7.9 ± 0.2** |
| N-MCT(30 mg/kg/day) | 1-7 | 4/10* | 11.2 ± 4.7** | 8.2 ± 0.1** |
| N-MCT(10 mg/kg/day) | 1-7 | 1/10 | 9/6 ± 2.6** | 8.3 ± 0.2** |
| Cidofovir (100 mg/kg/day) | 1-2 | 9/10*** | 8.0 | 7.0 ± 0.2** |
| Placebo | 1-7 | 1/20 | 7.9 ± 1.8 | 8.7 ± 0.1 |

| | | | | |
|---|---|---|---|---|
| a Twice daily (12 h apart) for N-MCT and once daily for cidofovir, starting 24 h after virus exposure. b Mean day of death ± SD of mice that died prior to day 21. ^{c} Log¹⁰ PFU/g ± SD determined on day 5 of the infection. * *P* < 0.5. ** *P* < 0.01. ** *P* < 0.001. | | | | |

The results of the experiments regarding activity of N-MCT against a severe vaccinia (IHD strain) respiratory infection in mice (high virus challenge of 10⁵ PFU/mouse) is presented in Table 7.

**Table 7.**

| Compound (mg/kg/day)^{a} | Treatment Days | Survivors/Total | MDD*^{b}* | Mean Lung Virus Titer*^{c}* |
|---|---|---|---|---|
| N-MCT (100 mg/kg/day) | 1-7 | 10/10*** | >21*** | 7.1 ± 0.3** |
| N-MCT(30 mg/kg/day) | 1-7 | 10/10*** | >21*** | 8.1 ± 0.1** |
| N-MCT(10 mg/kg/day) | 1-7 | 10/10*** | >21*** | 8.3 ± 0.1** |
| Cidofovir (100 mg/kg/day) | 1-2 | 10/10*** | >21*** | 4.7 ± 0.6** |
| Placebo | 1-7 | 4/20 | 8.2 ± 0.5 | 8.6 ± 0.3 |

| | | | | |
|---|---|---|---|---|
| a Twice daily (12 h apart) for N-MCT and once daily for cidofovir, starting 24 h after virus exposure. b Mean day of death ± SD of mice that died prior to day 21. ^{c}Log¹⁰ PFU/g ± SD determined on day 5 of the infection. * *P* < 0.5. ** *P* < 0.01. *** *P* < 0.001. | | | | |

### BALB/c Mice Inoculated with Cidofovir-Resistant Vaccinia WR Strain

The effect of twice daily i.p. treatment with N-MCT (compared to placebo or CDV) on the mortality of BALB/c mice challenged with a cidofovir-resistant vaccinia WR strain was investigated. N-MCT was prepared in 0.4 % CMC and delivered i.p. in 0.1 ml doses to treat the resulting infection. CDV was prepared in sterile saline and given i.p. in 0.1 ml doses. Animals were treated twice daily for 7 days beginning 1 day after viral challenge. Results of the testing (Table 8) indicated that N-MCT exhibited greater efficacy against the vaccinia WR strain than CDV, suggesting that N-MCT may be particularly well-suited for the treatment of strains of poxvirus resistant to conventional therapies.

**Table 8.**

| Treatment | Number of Survivors |
|---|---|
| Placebo - Saline | 0/20 |
| CDV (100 mg/kg/day) | 7/10 |
| N-MCT (100 mg/kg/day) | 10/10 |

### Results

N-MCT fully protected the infected mice from death resulting from infection by the vaccinia IHD strain at higher doses and was still significantly protective at the lowest dose tested. Its activity against cowpox was less dramatic compared to the case of vaccinia infection; however, significant protection was still observed at higher doses. In addition, the efficacy was dose-responsive. When tested against a more virulent vaccinia WR strain, N-MCT was not active. However, N-MCT was well tolerated by the mice even at the highest concentration tested (100 mg/kg) and N-MCT was active against a cidofovir (CDV)-resistant mutant strain, indicating its viral target may be different from that of cidofovir.

### Experiment #2

### Differences in Susceptibility of Pox Viruses to Antiviral Drugs

Both herpes simplex virus (HSV) and vaccinia virus (VV) express thymidine kinase (TK) activity and each of these enzymes can phosphorylate thymidine (see Kit, et al., 1967. J. Virol. 1, 238-240.; Kit, et a/., 1963. J. Mol. Biol. 6, 22-33). These enzymes differ in that the herpesvirus TK is a type I enzyme, whereas the VV TK is a type II enzyme (Black, et al., 1992. J. Biol. Chem. 267, 9743-9748). The HSV type I TK is the product of the of the *UL23* gene (McGeoch, et al., 1988. J. Gen. Virol. 69, 1531-1574), and like all type I enzymes, is active as a homodimer and lacks allosteric control (Jamieson, et al., 1974. J. Gen. Virol. 24, 481-492). This enzyme also exhibits a broad substrate specificity, including thymidine, 2'-deoxycytidine and synthetic nucleoside analogs (Coen, et al., 1980. Proc. Natl. Acad. Sci. U.S.A. 77, 2265-2269). VV TK is the prototypic type II enzyme and is encoded by the J2R gene (Hruby, et al., 1983. Proc. Natl. Acad. Sci. U.S.A. 80, 3411-3415). Like other members of this class, it is active as a homotetramer (Hruby, et al., 1982. J. Virol. 43, 403-409) and is allosterically inhibited by both dTTP and dTDP (Black, et al., 1992. J. Biol. Chem. 267, 6801-6806). The human TK is also a type II enzyme and like its homolog in VV, exhibits a restricted substrate specificity limited to thymidine and closely related analogs.

It is hypothesized that the observed differences in susceptibility to a number of antiviral drugs might be related to the distinct classes of TK enzymes expressed in these virus families. Thus, the poor activity of many nucleoside analogs against cowpox virus (CV) may reflect their inability to be activated by the type II TK expressed by this virus. To test this hypothesis, the antiviral activity of selected compounds against TK+ and TK- strains of both HSV-1 and CV was examined. Most of the compounds examined did not exhibit HSV-1 TK dependence. This is consistent with the notion that the poor phosphorylation of nucleoside analogs contributes significantly to the lack of activity for many compounds against orthopoxviruses. The approach described here to investigate TK dependence in CV can also be used to identify novel compounds that are preferentially activated by the orthopoxvirus TK.

### Cells and viruses

Primary human foreskin fibroblast (HFF) cells were prepared and passaged by methods described previously (Rybak, et al., 2000. Antimicrob. Agents Chemother. 44, 1506-1511). Vero cells were obtained from the American Type Culture Collection (Manassas, VA). The wild type (wt) HSV-1 strain F and TK⁻ strain DM2.1 were described and propagated as reported previously (Hartline, et al., 2005. Antiviral Res. 65, 97-105). CV strains delta crmA (TK⁺) and TK:GFP lacZ (TK⁻) were obtained from Pete Turner (University of Florida, Gainesville, FL) and were described previously (Ali, et al., 1994. Virology 202, 305-314). Cidofovir, idoxuridine (IDU), 5-bromodeoxyuridine (BrdU), vidarabine (AraA), 5-fluorodeoxyuridine (FdU), trifluridine (TFT), fialuridine (FIAU), fiacitabine (FIAC), sorivudine (BVAU), ACV and brivudin (BVDU) were either purchased (Sigma-Aldrich, St. Louis, MO) or were obtained through the NIAID, NIH, Bethesda, MD.

### CV β-galactosidase assay

Monolayers of Vero cells in 96-well plates were incubated at 37°C for 24 h in a humidified incubator. Drugs were then diluted in the plates and either TK⁺ or TK⁻ strains of CV were added at a multiplicity of infection of 0.05 PFU/cell. At 72 h post infection, the medium was removed and the β-galactosidase substrate, chlorophenol red-β-galactopyranoside, was added at a final concentration of 50 µg/ml in a phosphate-buffered saline solution. The conversion of the colorimetric substrate was determined by measuring the absorbance at 570 nm, and 50% effective concentration (EC₅₀) values were calculated. The TK⁻/TK⁺ EC₅₀ ratio was calculated in each experiment and an average ratio and standard deviation were calculated for all experiments.

### HSV-1 plaque reduction assay

HFF cells were seeded into six-well plates and incubated at 37°C. Two days later, drug was serially diluted 1:5 in MEM with 2% FBS using six concentrations of drug with a starting concentration of 100 µg/ml. Viruses were diluted in MEM containing 10% FBS to a concentration that yielded 20-30 plaques per well. The media were then aspirated from the wells and 0.2 ml of virus was added to each of triplicate wells with 0.2 ml of medium being added to control wells. The plates were then incubated for 1 h with shaking every 15 min and drug was added to appropriate wells. After an incubation period of 3 days, the cells were stained with 0.1 % crystal violet in 20% MeOH. The stain was aspirated, the wells washed with PBS and the plaques enumerated using a stereomicroscope. EC₅₀ values were calculated in a standard manner.

### Cytotoxicity assay

To determine the toxicity of drugs, HFF cells were seeded into 96-well plates at a concentration of 2.5×10⁴ cells/well in growth media. After 24 h, the media were aspirated and 100 µg/ml of drug was added to the first row of the plate and five-fold serial dilutions were performed. Following a 7 day incubation, the media were aspirated and 660 µg/ml of neutral red stain was added and incubated for 1 h. The monolayers were then washed and the dye was dissolved in a solution containing 50% ethanol and 1% glacial acetic acid. The plates were mixed for 15 min on a rotating shaker and the optical densities were determined at 550 nm. CC₅₀ values were interpolated from the data.

### Genetic resistance assays

The principle behind both the CV and HSV-1 genetic resistance assays is the same. The efficacy of antiviral drugs was determined in TK⁺ and TK⁻ strains of the same virus and the ratio of the EC₅₀ against the TK⁻ virus to the EC₅₀ against the TK⁺ virus was used as a measure of TK dependence. The nucleotide analog cidofovir (CDV) was used as a negative control for both these viruses. As positive controls, acyclovir (ACV) and 5-iododeoxyuridine (IdU) were used in HSV-1 and CV assays, respectively, since these drugs require phosphorylation for their antiviral activity.

### Amino acid alignment and phylogeny

Amino acid sequences for TK homologs were downloaded from GenBank and subjected to a clustal W alignment and unrooted phylogenetic trees were constructed using Vector NTI (Invitrogen, San Diego, CA). Gene ID numbers in the analysis and abbreviations used are VV, 29692200; HSV-1, 9629403; HSV-2, 9629292; *Homo sapiens* (human), 4507519; goatpox virus, 55274605; VZV, 118822; EBV, 23893647; variola virus, 66679; fowlpox virus, 221413; monkeypox virus, 22096356; CV, 20178469.

### Results

A set of antiviral drugs was selected based on previously described activity against HSV-1 and orthopoxviruses. Since IDU has been used in the laboratory to select for TK-deficient orthopoxviruses (Byrd, et al., 2004. Methods Mol. Biol. 269, 31-40) and a number of analogs have good activity against VV (De Clercq, E., 1980. Methods Find. Exp. Clin. Pharmacol. 2, 253-267), additional analogs were studied to determine if this gene was involved in the activation of other compounds in this series. The structures of these compounds were as follows:

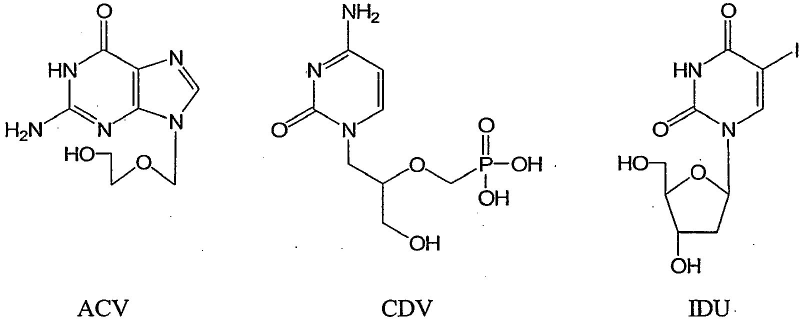

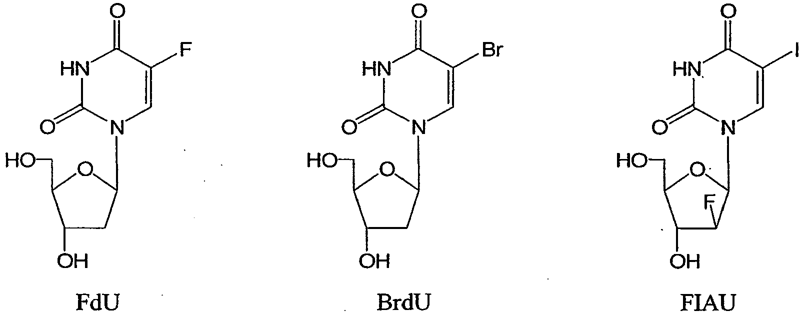

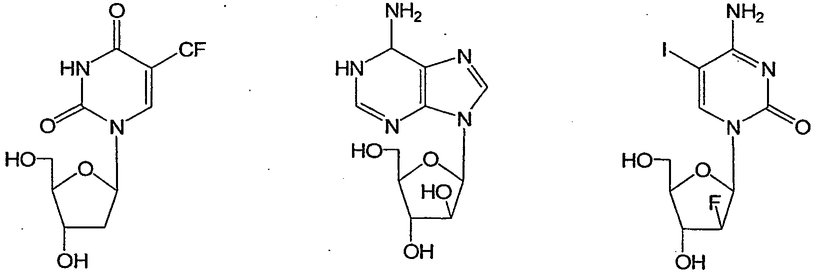

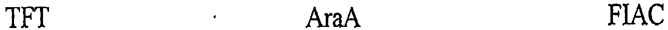

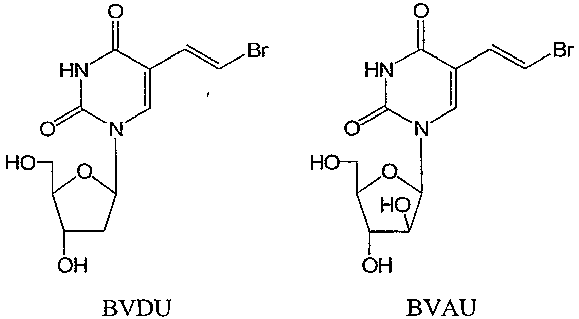

A total of 11 compounds including control drugs were tested in the genetic resistance assays and the EC₅₀ ratios were calculated. Results for antiviral activity of the compounds against TK⁺ and TK⁻ strains of HSV-1 and CV are presented in Table 9.

**Table 9.**

| | HSV-1 | | | CV | | | Toxicity^{d} CC₅₀ |
|---|---|---|---|---|---|---|---|
| | F^{a} EC₅₀ (TK+) | DM2.1^{a} EC₅₀ (TK-) | EC₅₀ ratio^{b} | delta crmA^{c} EC₅₀ (TK⁺) | TK:GFP^{c} lacz EC₅₀ (TK⁻) | EC₅₀ ratio^{b} | |
| ACV | 0.35 ± 0.2 | >100 ± 0 | >286 | >30 | >30 | 1 | >100 ± 0 |
| CDV | 1.5 ± 1.1 | 1.5 ± 1.4 | 1 | 1 6.1 ± 0.30 | 3.3 ± 2.3 | 0.54 | >100 ± 0 |
| IDU | 2.1 ± 0.1 | 54 ± 8.1 | 26 | 2.2 ± 0.87 | 16 ± 9.3 | 7.3 | >100 ± 0 |
| AraA | 6.4 ± 3.4 | 4.9 ± 0 | 0.78 | 0.91 ± .005 | 0.78 ± 0.2 | 0.86 | >100 ± 0 |
| BrdU | 1.6 ± 0.2 | 39 ± 6.3 | 24 | 0.37a ± 0 | 19a ± 15 | 51 | >100 ± 0 |
| FdU | 2.5 ± 1.7 | 4.5 ± 3.3 | 1.8 | 0.37a ± 0 | 0.37a ± 0 | 1 | 60 ± 0.7 |
| TFT | 1.0 ± 0.2 | 1.1 ± 0.3 | 1.1 | 0.42 ± 0.33 | 0.72 ± 0.28 | 1.7 | >100 ± 0 |
| FIAU | 0.05 ± 0.03 | 3.9 ± 3.3 | 78 | 14 ± 7.0 | 19 ± 6.7 | 1.4 | >100 ± 0 |
| FIAC | 0.06 ± 0.02 | 8.0 ± 1.3 | 133 | 18 ± 0.25 | 9.2 ± 3.7 | 0.51 | >100 ± 0 |
| BVDU | 0.09 ± 0 | 100 ± 0 | 1111 | >30 | >30 | 1 | >100 ± 0 |
| BVAU | 0.05 ± 0.02 | >100±0 | >2000 | >30 | >30 | 1 | >100 ± 0 |

| | | | | | | | |
|---|---|---|---|---|---|---|---|
| ^{a} Average of two experiments ± standard deviation (µg/ml). ^{b} Ratio of EC₅₀ values for TK⁻ and TK⁺ strains of the virus. ^{c} Average of five experiments ± standard deviation. ^{d} Determined by duplicate neutral red uptake assays. | | | | | | | |

For HSV-1, the wt strain was very sensitive to ACV, while the TK⁻ strain was highly resistant to the drug and yielded an EC₅₀ ratio of 286. In contrast, EC₅₀ values for the CDV negative control were indistinguishable between these two strains yielding an EC₅₀ ratio of 1, confirming that the assay could identify compounds that require TK in their mechanism of action. TFT, FdU and AraA did not appear to be selectively phosphorylated to a significant extent by the HSV-1 TK since the EC₅₀ ratios were very close to 1, but each of the remaining compounds was significantly less effective against the TK⁻ virus suggesting not only that this enzyme was required for their activation. Among these, ACV, BVAU, BVDU, FIAC, and FIAU appeared to be the most dependent on this enzyme suggesting that they are not phosphorylated to a significant extent by host kinases. IDU and BrDU were dependent on TK, but retained some activity in the TK⁻ virus suggesting that they might be activated to some degree by enzymes other than the HSV-1 TK (type 1).

This same set of compounds was also tested against CV, and EC₅₀ ratios were calculated to assess the ability of the type II TK from this virus to activate these same compounds (Table 9). Both strains of CV were fully sensitive to the CDV control and yielded an EC₅₀ ratio of 0.54. The TK⁺ strain of CV was sensitive to IDU as reported previously for CV (Kern, E.R., 2003. Antiviral Res. 57, 35-40; Smee, et al., 2004. Nucleosides, Nucleotides Nucleic Acids 23, 375-383) and vaccinia virus (Kern, E.R., 2003. Antiviral Res. 57, 35-40; Neyts, et al., 2002. Antimicrob. Agents Chemother. 46, 2842-2847), while the TK⁻ CV strain was significantly less sensitive to the drug and yielded an EC₅₀ ratio of 7.3. These results indicated that the assay was capable of identifying compounds that require TK for their activity. The closely related analog, BrdU, was also much less effective against the TK⁻ virus and yielded a TK⁻/TK⁺ EC₅₀ ratio of >50. Both TFT and FdU were equally effective in inhibiting both strains of CV and HSV-1, as was expected since both these molecules are inhibitors of thymidylate synthetase (Emura, et al., 2004. Int. J. Mol. Med. 13, 249-255). The antiviral activity of AraA was also independent of TK and is consistent with results obtained with HSV-1. ACV, BVDU, and BVAU did not significantly inhibit replication of either CV strain, suggesting that either they are not activated by the CV-encoded TK or they are not substrates for the viral DNA polymerase. Both FIAC and FIAU exhibited modest activity against TK⁻ and TK⁺ strains of the virus suggesting that the compounds can inhibit the CV DNA polymerase, but also that the TK expressed by CV does not appear to activate the compounds to a measurable extent. This contrasts with the high EC₅₀ ratios for FIAC and FIAU in HSV-1 of 78 and 133, respectively.

To confirm results from the TK dependence studies, enzyme kinetics were determined for N-MCT and other substrates using purified VV TK in enzymatic assays. Enzyme kinetic parameters for substrates of VV TK are provided in Table 10. Several thymidine analogs, including N-MCT had *Kₘ* values that were comparable to thymidine suggesting that they were good substrates for the enzyme. Values for *Vₘₐₓ* were also obtained and were used to calculate the efficiency (*Vₘₐₓ*/*Kₘ*) for each of the substrates. These results suggested that N-MCT was efficiently phosphorylated by the enzyme.

**Table 10.**

| Substrate | *Kₘ* (µM)^{a} | *Vₘₐₓ* (µMmin⁻¹ mg⁻¹) | *Vₘₐₓ*/*Kₘ* |
|---|---|---|---|
| dThd | 49 ± 7.6 | 289 ± 137 | 5.9 |
| BrdU | 30 ± 6.2 | 281 ± 59 | 9.4 |
| IdU | 31 ± 4.7 | 222 ± 58 | 7.2 |
| FdU | 113 ± 85 | 108 ± 63 | 0.96 |
| TFT | 14 ± 4.3 | 333 ± 44 | 24 |
| FIAU | 4.3 ± 2.5 | 99 ± 41 | 22.7 |
| N-MCT | 32 ± 14 | 114 ± 23 | 3.6 |
| FIAC | >135 | <0.14 | <0.001 |
| CDV | >158 | <0.025 | <0.0002 |

| | | | |
|---|---|---|---|
| *^{a}* Average of four or more determinations with standard deviations as shown. | | | |

The effect of HSV-1 TK and CV TK on the antiviral activity of several related nucleoside analogs was tested. In these assays, a positive result is significant in that it suggests that TK is involved in the mechanism of action of the drug, and also implies selectivity since viral TK gene must be dominant over cellular kinases. It does not demonstrate the phosphorylation of the drugs directly, but suggests that it may be involved given the kinase activity of the enzymes. There are many potential explanations for negative results including the possibility that both cellular and viral enzymes could be activating the drug. Closely related nucleoside analogs were selected because the orthopoxviruses express type II TK enzymes and were expected to have a narrow substrate specificity.

Results presented were consistent with this prediction since very few analogs of IDU appear to be activated by CV TK. The narrow substrate specificity of this enzyme was apparent even with the small set of compounds tested. As expected, IDU was dependent on the CV TK and the substitution of bromine for iodine was well tolerated by the enzyme as evidenced by the high TK⁻/TK⁺ EC₅₀ ratio of 51. However, the substitution of a larger bromovinyl group at this same position eliminated its activity against both strains of CV and this is consistent with a lack of activation by CV TK, but it is also possible that the triphosphate metabolite of this compound did not inhibit the CV DNA polymerase.

The introduction of either fluorine or a trifluoromethyl group at the C-5 position makes FdU and TFT inhibitors of thymidylate synthetase and predictably, both these molecules inhibit TK⁺ and TK⁻ strains of both HSV-1 and CV. Although TFT does not exhibit TK dependence in HSV, the viral enzyme can phosphorylate the drug to a limited extent and the monophosphorylated drug can be detected in TK⁻ cells (Field, et al., 1981. J. Infect. Dis. 143, 281-285).

Substitutions on the sugar moiety also appear to be poorly tolerated since the addition of a fluorine at the 2' position in FIAU rendered it equally effective against both strains of CV. Results from FIAC and FIAU were particularly informative since both these drugs exhibit modest antiviral activity against CV, and this is consistent with the compounds inhibiting the DNA polymerase. However, neither of these compounds appeared to be activated by the type II TK encoded by this virus and contrasts with results in HSV-1, where both these compounds were efficiently phosphorylated by the type I TK encoded by this herpesvirus. The fact that the relative potency of these compounds against CV is similar to that against the TK⁻ strain of HSV-1 is also consistent with this interpretation of the data.

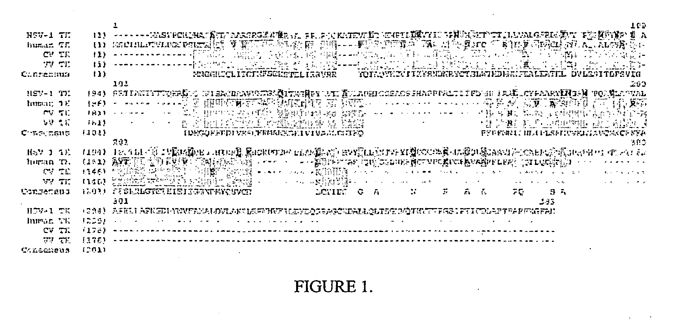

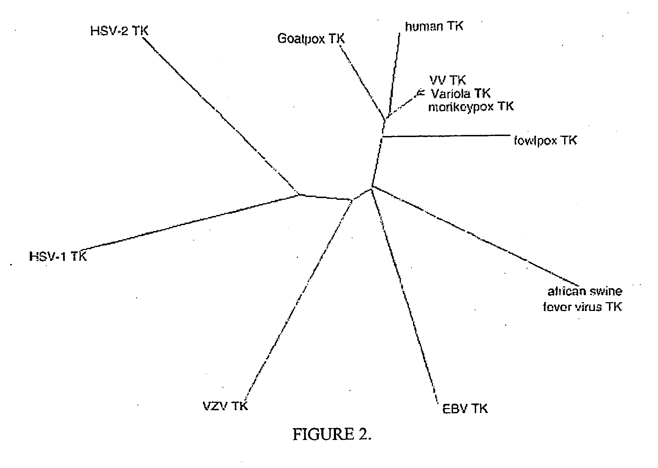

The results obtained confirm that the CV TK has a rather limited substrate specificity compared to the TK encoded by HSV-1. This outcome was expected since this virus encodes a type II TK homolog that is more closely related to the human enzyme than the enzyme from HSV-1 (Figure 1). TK homologs encoded by VV, CV, variola virus and monkeypox virus are all quite similar compared to the high degree of sequence divergence in the type I enzymes encoded by the herpesviruses (Figure 2). The high degree of similarity among the human orthopoxviruses TK homologs is important and suggests that the assay reported will be predictive for variola virus and monkeypox virus. The high degree of amino acid identity among the orthopoxvirus enzymes and the human homolog as well as the narrow substrate specificity that is characteristic of type II enzymes will likely make it difficult to identify nucleoside analogs that are only activated by these enzymes. Nevertheless, it should be possible to identify nucleosides that are selectively phosphorylated by the orthopoxvirus TK homolog (Figure 2). Once activated in cells infected with CV, cellular enzymes could phosphorylate the compounds to the level of the triphosphate to make them inhibitors of the viral DNA polymerase. The CV genetic resistance assay described herein is capable of identifying inhibitors that depend on the viral TK for their activity.

TDNA polymerase inhibitors were developed that are specifically activated by the type I TK molecules encoded by the herpesviruses, and antiviral drugs with this same mechanism of actions can be developed against orthopoxviruses. Specificity derived through selective phosphorylation is a highly effective strategy for the treatment of orthopoxvirus infections, and compounds that act by such a mechanism can be developed by identifying drugs that require the viral TK for their activity. Compounds thus identified are expected to have a relatively good toxicity profile since, if they remain unphosphorylated, they are unlikely to inhibit cellular polymerases, and thus will be useful new therapies for the treatment of orthopoxvirus infections.

### Experiment #3

### Antiviral Activity of N-MCT Against Orthopoxviruses

The antiviral activity of N-MCT against the orthopoxviruses was confirmed and the function of TK in the mechanism of action of this compound was investigated. These studies suggested that this molecule was specifically phosphorylated by the TK homologs in both CV and HSV-1 and that the active metabolite inhibited viral replication at the level of viral DNA synthesis. These data taken together with the pharmacokinetic properties of the drug (Noy, et al. 2002. Cancer Chemother. Pharmacol. 50:360-366) suggested that it might exhibit antiviral activity *in vivo*. N-MCT was tested in a murine model against VV, CV, and HSV-1. Mice treated intraperitoneally (i.p.) with N-MCT beginning 24 h after infection were protected from lethality with each of these viruses at relatively low doses of drug. N-MCT is a molecule that can be activated by the divergent TK homologs in orthopoxviruses and some herpesviruses and thus can be useful in therapy for these infections in humans.

### Cells and Viruses

Methods for producing and passaging human foreskin fibroblast (HFF) cells were described previously (Rybak, et al. 2000. Antimicrob. Agents Chemother. 44:1506-1511). Culture medium for all cell lines was minimal essential medium (MEM) containing 10% fetal bovine serum (FBS) and standard concentrations of L-glutamine, penicillin, and gentamicin. VV strains WR, Copenhagen, and IHD were obtained from the American Type Culture Collection (ATCC; Manassas, Va.). Working stocks of these viruses were propagated in Vero cells obtained from the ATCC. CV, strain Brighton, was provided by John W. Huggins (Department of Viral Therapeutics, Virology Division, U.S. Army Medical Research Institute of Infectious Disease, Frederick, MD). *ΔcrmA* (TK⁺) and TK:GFP *lacZ* (TK⁻) CV strains were obtained from Pete Turner (University of Florida, Gainesville, FL) and were described previously (Ali, et al. 1994. Virology 202:305-314): The wild-type HSV-1 strain F and TK⁻ strain DM2.1 were described and propagated as reported previously (Hartline, et al. 2005. Antivir. Res. 65:97-105). CDV (Vistide) was provided by Gilead Pharmaceuticals (Foster City, CA), and N-MCT and the other compounds were obtained through the NIAID, NIH, Bethesda, MD.

### VV, CV, and HSV plaque reduction assays

For VV and CV, HFF cells were added to six-well plates and incubated for 2 days at 37°C with 5% CO₂ and 90% humidity. On the day of assay, drug at two times the final desired concentration was diluted serially 1:5 in 2× MEM with 10% FBS to provide six concentrations. Aspiration of culture medium from triplicate wells for each drug concentration was followed by addition of 0.2 ml per well of diluted virus, which would give 20 to 30 plaques per well in MEM containing 10% FBS or 0.2 ml medium for drug toxicity wells. The plates were incubated for 1 h with shaking every 15 min. An equal amount of 1% agarose was added to an equal volume of each drug dilution, and this mixture was added to each well in 2 ml volumes and the plates were incubated for 3 days. The cells were stained with a solution of neutral red in phosphate-buffered saline (PBS) and incubated for 5 to 6 h. The stain was aspirated, plaques were counted using a stereomicroscope at 10× magnification, and 50% effective concentrations (EC₅₀s) were calculated by standard methods. The HSV plaque reductions were essentially the same as those for VV and CV with the following changes. The drug solutions were prepared at the desired concentration in MEM with 2% FBS, and a liquid overlay with pooled human serum containing antibodies to HSV instead of agarose was used. At 72 h following infection, the medium containing the drug was aspirated and the monolayers were stained with 1 ml of a solution of 0.01 % crystal violet in 60% methanol for 10 min. Residual stain was then washed from the wells with 1 ml PBS, and plaques were counted.

### CV β-galactosidase assay

Monolayers of HFF cells in 96-well plates were incubated at 37°C for 24 h in a humidified incubator. Drugs were then diluted in the plates, and either TK⁺ or TK⁻ strains of CV were added at a multiplicity of infection of 0.05 PFU/cell (Prichard, et al. Distinct thymidine kinases encoded by cowpox virus and herpes simplex virus contribute significantly to the differential antiviral activity of nucleoside analogs. Antivir. Res., in press). At 48 h postinfection, the medium was removed and the β-galactosidase substrate chlorophenol red-β-galactopyranoside was added at a final concentration of 50 µg/ml in PBS. The conversion of the colorimetric substrate was determined by measuring the absorbance at 570 nm, and EC₅₀s were calculated by standard methods (Prichard, et al. 1990. Antivir. Res. 14:181-205). The EC₅₀ ratio for TK⁺ and TK⁻ viruses was calculated and used as a measure of TK dependence.

### Cytotoxicity determination

The neutral red uptake assay was conducted as reported previously (Keith, et al. 2003. Antimicrob. Agents Chemother. 47:2193-2198). Briefly, HFF cells were plated into 96-well plates at a concentration of 2.5×10⁴ cells per well. After 24 h, the medium was aspirated and 125 µl of each drug concentration in MEM with 2% FBS was added to the first row of wells in triplicate. Serial 1:5 dilutions were performed using the Beckman BioMek liquid handling system. After compound addition, the plates were incubated for 7 days in a CO₂ incubator at 37°C. After incubation, the medium/drug was aspirated and 200 µl/well of 0.01 % neutral red in PBS was added and incubated for 1 h. The dye was aspirated, and the cells were washed with PBS using a Nunc plate washer. After the PBS was removed, 200 µl/well of a solution containing 50% ethanol and 1% glacial acetic acid was added. The plates were placed on a rotary shaker for 15 min, and the optical densities were determined at 540 nm. The concentration of drug that reduced cell viability by 50% was then calculated. In the proliferation assay, cells were seeded in six-well plates at a concentration of 2.5×10⁴ cells/ml. After 24 h, the medium was aspirated and drug serially diluted 1:5 was added to the appropriate wells. The cells were incubated for 72 h at 37°C and then trypsinized and counted using a Coulter counter. Standard methods were used to determine the drug concentration which inhibited cell proliferation by 50%.

### DNA synthesis assay

Drugs were diluted to final concentrations of 30, 10, 3, 1, and 0.3 µg/ml and added to confluent monolayers of HFF cells in six-well plates. The WR strain of VV was used to infect the cells at a multiplicity of infection of 0.5 PFU/cell. Total DNA was extracted 24 h postinfection by lysing the cells in a buffer containing 10 mM Tris, pH 7.4, 1 mM EDTA, and 1% sodium dodecyl sulfate and incubating them with 100 µg of proteinase K for 60 min at 37°C. DNA samples were purified using QIAGEN's QiaQuick PCR purification system according to the manufacturer's protocol. Samples were cleaved with EcoRV, and fragments were separated on an agarose gel, transferred to a nylon membrane, and hybridized to a digoxigenin-labeled probe (Roche Applied Science, Indianapolis, IN), specific for VV DNA (coordinates 128257 to 129537 in AY243312).

### Activity of N-MCT in mice inoculated with HSV, VV, or CV.

Female BALB/c mice, 3 to 4 weeks of age, were obtained from Charles River Laboratories (Raleigh, NC). Mice were group housed in microisolator cages, and each treatment group contained 15 mice. Mice were obtained, housed, utilized, and euthanized according to policies of the USDA and AAALAC. All animal procedures were approved by the University of Alabama at Birmingham Institutional Animal Care and Use Committee prior to initiation of studies. Infections were initiated by intranasal (i.n.) inoculation of BALB/c mice. Mice were infected with an approximate 90% lethal dose of HSV-1 strain E377 (1.0×10⁵ PFU/animal), CV strain BR (3.3×10⁴ PFU/animal), VV strain WR (1.0×10⁴ PFU/animal), or VV strain IHD (2.5×10⁴ PFU/animal) using a micropipettor and a total volume of 40 µl per animal. N-MCT was suspended in 0.4% carboxymethyl cellulose (CMC) to yield 50-, 16.7-, or 5.6-mg/kg-of-body-weight doses in a volume of 0.1 ml. Mice were treated i.p. with N-MCT twice daily, approximately 12 h apart beginning 24 h post-viral inoculation with a 5-day duration of therapy for orthopoxviruses and a 7-day duration for HSV-1. Vehicle-treated mice were included as negative controls, and either ACV or CDV was included as a positive control. Mortality rates were analyzed by Fisher's exact test, and mean day of death was analyzed by Mann-Whitney U rank sum. A *P* value of 0.05 or less was considered significant.

### Results

The antiviral activity of N-MCT was characterized and N-MCT was evaluated for its potential as a therapy for orthopoxvirus and HSV infections. Efficacy and cytotoxicity of N-MCT was determined in HFF cells infected with the Copenhagen strain of VV and the Brighton strain of CV (Table 11).

**Table 11.**

| Drug | VV (Copenhagen) | | CV (Brighton) | | CC₅₀^{a} | IC₅₀^{b} |
|---|---|---|---|---|---|---|
| | EC₅₀^{c} | SI*^{d}* | EC₅₀^{c} | SI*^{d}* | | |
| N-MCT | 0.55 ± 0.13 | >182 | 1.5 ± 1.2 | >67 | >100 ± 0 | 29 ± 5.4 |
| CDV | 3.3 ± 0.3 | >30 | 4.4 ± 0.6 | >23 | >100 ± 0 | 20 ± 7 |

| | | | | | | |
|---|---|---|---|---|---|---|
| *^{a}* CC₅₀, 50% cellular cytotoxicity determined by neutral red uptake in stationary monolayers of HFF cells. *^{b}* IC₅₀, 50% inhibition of cell proliferation. *^{c}* Average of two or more assays shown in units of µg/ml with standard deviations. *^{d}* SI, selective index calculated as the CC₅₀ divided by the EC₅₀. | | | | | | |

In these standard plaque assays, the drug yielded EC₅₀s against both viruses that were lower than those for the CDV control. Cytotoxicity was also determined in both a neutral red uptake assay and a proliferation assay and appeared to be at least comparable to that of CDV, resulting in selective indices that were higher than those for CDV.

Previous reports suggested that N-MCT was a substrate of the HSV-1 TK, since an inhibitor of this enzyme reduced the phosphorylation of the drug (Zalah, et al. 2002. Antivir. Res. 55:63-75), and the enzyme supplied in *trans* conferred sensitivity to the drug in uninfected cells (Schelling, et al. 2004. J. Biol. Chem. 279:32832-32838). It is hypothesized that the type II TK homologs expressed by orthopoxviruses might also be capable of activating this compound, since its structure was very close to the natural substrate of the enzyme. The antiviral activity of N-MCT against TK⁺ and TK⁻ strains of CV and HSV-1 in a genetic resistance assay was determined (Prichard, et al. Distinct thymidine kinases encoded by cowpox virus and herpes simplex virus contribute significantly to the differential antiviral activity of nucleoside analogs. Antivir. Res., in press). Drugs that require TK for their activation exhibit elevated TK⁻/TK⁺ EC₅₀ ratios, whereas compounds that do not require the enzymes to be active yield EC₅₀ ratios near unity. In HSV-1, ACV was used as a positive control since it required phosphorylation by the TK and CDV was used as a negative control since it does not require phosphorylation by the enzyme to be active. The high EC₅₀ ratios for ACV and idoxuridine (IDU) reflect the fact that they require phosphorylation by this enzyme (Table 12).

**Table 12.**

| Drug | EC₅₀ (µg/ml) | | | | | |
|---|---|---|---|---|---|---|
| | CV (Δ*crm*) *lacZ*) TK⁺ | CV (*TK:gpf lacZ*) TK⁺ | Ratio^{b} | HSV-1 (F) TK⁺ | HSV-1 (DM2.1) TK⁻ | Ratio^{b} |
| N-MCT | 2.0 ± 2.0^{a} | 28 ± 2.3 | 14 | 0.07 ± 0.02 | 6.3 ± 3.9 | 90 |
| CDV | 3.5 ± 3.3 | 4.2 ± 3.9 | 1.2 | 1.5 ± 1.1 | 1.5 ± 1.4 | 1.0 |
| ACV | NA*^{c}* | NA | NA | 0.3 ± 0 | >100 ± 0 | 333 |
| IDU | 0.4 ± 0.18 | 22 ± 9.3 | 55 | 2.1 ± 0.1 | 54 ± 8.1 | 26 |

| | | | | | | |
|---|---|---|---|---|---|---|
| *a* Average of three or more experiments with standard deviation. *b* EC₅₀ ratio for TK⁻ and TK⁺ viruses, respectively. *c* NA, not applicable. | | | | | | |

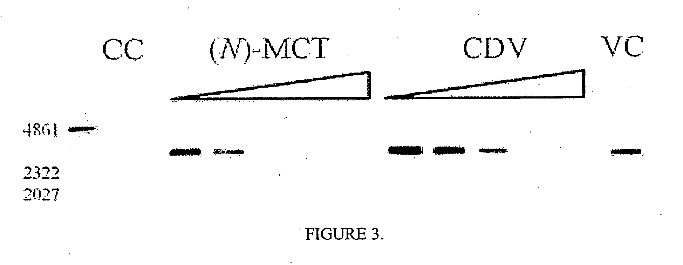

N-MCT also yielded an elevated EC₅₀ ratio, confirming that it was phosphorylated by TK, while the CDV negative control was unaffected by the deletion of this gene. In CV, IDU was used as a positive control since it was known to be activated by its TK, and CDV was used as a negative control. CDV was equally effective against the two viruses and yielded an EC₅₀ ratio of 1.2, while IDU was much less effective against the TK⁻ strain of CV and yielded an EC₅₀ ratio of 55 (Table 12). The TK⁻ virus was also significantly less sensitive to N-MCT and yielded an EC₅₀ ratio of 14, suggesting that this compound may also require TK for its activation. The ability of this compound to inhibit DNA synthesis was determined using CDV as a control. N-MCT was at least as effective in inhibiting VV DNA synthesis as the CDV positive control and yielded EC₅₀s of 1 and 3 µg/ml, respectively (Figure 3). Both of these values were comparable to the antiviral EC₅₀s of 0.6 and 3 µg/ml, respectively, and were sufficient to explain the antiviral effects of the drug. The antiviral activity of the compound *in vitro* taken together with its pharmacokinetic parameters (Noy, et al. 2002. Cancer Chemother. Pharmacol. 50:360-366) and the mechanism of action studies suggest that it should be an effective and highly selective compound *in vivo.* An established animal model was used to evaluate the antiviral activity of the compound against HSV-1 (Kern, et al. 1973. J. Infect. Dis. 128:290-299). BALB/c mice were infected i.n. with the E377 strain of HSV-1, and animals were treated twice daily beginning 24 h after infection. In this experiment, 7 of 14 animals succumbed to the infection, suggesting that the inoculum was slightly lower than the 90% lethal dose target dose. Nevertheless, all doses of the drug protected mice from mortality (*P* < 0.01) and were comparable to the ACV positive control (Table 13).

**Table 13.**

| Treatment*^{a}* | Mortality | | | MDD*^{b}* | *P* value |
|---|---|---|---|---|---|
| | No. dead / total no. | % | *P* value | | |
| Placebo | | | | | |
| 0.4% CMC | 7/14 | 50 | | 8.0 | |
| ACV | | | | | |
| 50 mg/kg | 0/15 | 0 | <0.01 | | |
| 16.7 mg/kg | 0/15 | 0 | <0.01 | | |
| 5.6 mg/kg | 0/15 | 0 | <0.01 | | |
| N-MCT | | | | | |
| 50 mg/kg | 0/15 | 0 | <0.01 | | NS^{c} |
| 16.7 mg/kg | 0/15 | 0 | <0.01 | | NS |
| 5.6 mg/kg | 1/15 | 7 | <0.05 | 20 | 0.05 |

| | | | | | |
|---|---|---|---|---|---|
| *^{a}* N-MCT was prepared in 0.4% CMC and delivered i.p. twice daily in 0.1-ml doses. ACV was prepared in sterile water and given i.p. twice daily in 0.1 ml doses. All animals were treated for 7 days beginning 24 h postinfection. *^{b}* MDD, mean day of death. *^{c}* NS, not significant. | | | | | |

These data suggest that this compound can be useful in the treatment of HSV infections. Established models of orthopoxvirus infections were also used to evaluate the efficacy of this compound against lethal VV and CV infections in BALB/c mice (Quenelle, et al. 2004. Antimicrob. Agents Chemother. 48:404-412). In the first study, groups of 15 animals were infected i.n. with a lethal dose of the IHD strain of VV. Drug was administered i.p. twice daily at doses of 50, 16.7, and 5.6 mg/kg starting 24 h post-i.n. inoculation. Mortality in all mice treated with vehicle was 100%, whereas all mice treated with CDV at 15 mg/kg given once daily survived (Table 14).

**Table 14.**

| Strain and treatment*^{a}* (mg/kg) | | | Mortality | | | MDD*^{b}* | *P* value |
|---|---|---|---|---|---|---|---|
| | | | No. dead / total no. | % | *P* value | | |
| VV strain IHD | | | | | | | |
| | 0.4% CMC | | 15/15 | 100 | | 7.6 | |
| | CDV | | | | | | |
| | | 15 | 0/15 | 0 | <0.001 | | |
| | N-MCT | | | | | | |
| | | 50 | 0/15 | 0 | <0.001 | | |
| | | 16.7 | 0/15 | 0 | <0.001 | | |
| | | 5.6 | 3/15 | 20 | <0.001 | 8.7 | <0.05 |
| VV strain WR | | | | | | | |
| | 0.4% CMC | | 15/15 | 100 | | 7.6 | |
| | CDV | | | | | | |
| | | 15 | 0/15 | 0 | <0.001 | | |
| | N-MCT | | | | | | |
| | | 50 | 0/15 | 0 | <0.001 | | |
| | | 16.7 | 2/15 | 13 | <0.001 | 7.5 | NS*^{c}* |
| | | 5.6 | 12/15 | 80 | NS | 8.2 | NS |
| CV strain Brighton | | | | | | | |
| | 0.4% CMC | | 15/15 | 100 | | 7.6 | |
| | CDV | | | | | | |
| | | 15 | 0/15 | 0 | <0.001 | | |
| | N-MCT | | | | | | |
| | | 50 | 2/15 | 13 | <0.001 | 7.5 | NS |
| | | 16.7 | 3/15 | 20 | <0.001 | 13.3 | 0.01 |
| | | 5.6 | 6/15 | 40 | <0.001 | 14.0 | 0.05 |

| | | | | | | | |
|---|---|---|---|---|---|---|---|
| *^{a}* N-MCT was prepared in 0.4% carboxymethyl cellulose and delivered i.p. twice daily in 0.1-ml doses. CDV was prepared in sterile saline and given i.p. once daily in 0.1-ml doses. All animals were treated for 5 days beginning 24 h postinfection. *^{b}* MDD, mean day of death. *^{c}* NS, not significant. | | | | | | | |

All animals treated with 50 or 16.7 mg/kg N-MCT administered twice daily survived, and 80% survived at the 5.6 mg/kg dose. All treatments resulted in significantly improved mortality compared to that of vehicle treated controls (*P* < 0.001). The successful treatment of the IHD strain of VV prompted us to repeat the experiment using the WR strain, which is a more pathogenic strain in our experimental model and has also been reported to be more lethal by others (Smee, et al. 2004. Int. J. Antimicrob. Agents 23:430-437). All mice survived when treated with CDV at 15 mg/kg, and mortality was 100% in vehicle-treated control mice (Table 14). All animals treated with N-MCT at a dose of 50 mg/kg survived the infection, and mortality was 13% and 80% at the 16.7 and 5.6 mg/kg dose regimens given twice daily, respectively. In this experiment the drug significantly protected mice at the high and medium dose (*P* < 0.001) but there was no significant protection at the low dose. These data are consistent with the first experiment and also with the notion that the WR strain is a slightly more virulent virus in this model. The same experiment was repeated against the Brighton strain of CV to confirm that N-MCT was active against another orthopoxvirus. In this experiment, the CDV control protected all animals from lethal infection with CV and all vehicle-treated animals died (Table 14). Animals treated at all doses of the test drug were significantly protected from mortality (*P* < 0.001), although a few animals died in each of the three treatment groups. These data taken together indicate that N-MCT is highly effective in treating orthopoxvirus infections *in vivo.*

Antiviral activity of N-MCT against HSV-1, VV, and CV both *in vitro* and *in vivo* was demonstrated. This compound is unusual in that it exhibits TK dependence both in a herpesvirus and in an orthopoxvirus, and its rather uncommon spectrum of activity appears to reflect its selective phosphorylation in cells infected with these viruses. While many nucleoside analogs can be activated by the type I TK homologs encoded by some of the herpesviruses, few are apparently phosphorylated by the distinct type II TK molecules expressed by orthopoxviruses and including IDU, bromodeoxyuridine (De Clercq, E. 2001. Clin. Microbiol. Rev. 14:382-397), and N-MCT as described herein. A previous report suggested that the metabolism of this compound required the enzymatic activity of the HSV-1 TK and that the compound was converted to N-MCT triphosphate, which was incorporated in cellular DNA by the host polymerase (Marquez, et al. 2004. J. Am. Chem. Soc. 126:543-549). The data suggest that the compound may inhibit both orthopoxvirus and herpesvirus DNA polymerases and impair viral DNA synthesis by a similar mechanism. Alternative interpretations of the data exist, and it is possible that N-MCT interferes with orthopoxvirus DNA synthesis indirectly through the inhibition of thymidylate synthetase by the monophosphate form of the drug. It is also possible, albeit unlikely, that differences in drug activities against CV are caused by genetic differences outside the TK locus. Although additional experiments will be required to confirm directly the inhibition of the VV DNA polymerase by N-MCT triphosphate, the active site is highly conserved between this enzyme and its homolog in herpes simplex virus. This proposed mechanism of action appears to be sufficient to explain the spectrum of activity of this compound. It is active against the orthopoxviruses and the alphaherpesviruses since they express enzymes with TK activity. The gammaherpesvirus Epstein-Barr virus also encodes a TK homolog, and the drug inhibits its replication with an EC₅₀ of 0.45 µg/ml (data not shown). Human herpesvirus 8 also expresses a TK homolog and also appears to be sensitive to the drug (Zhu, et al. 2005. Antimicrob. Agents Chemother. 49:4965-4973). In contrast, this compound appears to be inactive against the betaherpesviruses (human cytomegalovirus and human herpesvirus 6) which do not express an enzyme with TK activity, an observation which is also consistent with this mode of action. The property of TK dependence is significant since it should confer the desirable characteristics of good antiviral activity and minimal toxicity *in vivo.* N-MCT is a compound with an interesting spectrum of activity that is conferred by its selective phosphorylation by some herpesvirus and orthopoxvirus TK homologs, and can be used to treat both herpesvirus and orthopoxvirus infections.

Methods and compositions that are suitable for use in conjunction with aspects of the preferred embodiments are disclosed in U.S. Patent No. 5,840,728; U.S. Patent No. 5,629,454; and U.S. Patent No. 5,869,666.

The term "comprising" as used herein is synonymous with "including," "containing," or "characterized by," and is inclusive or open-ended and does not exclude additional, unrecited elements or method steps.

All numbers expressing quantities of ingredients, reaction conditions, and so forth used in the specification are to be understood as being modified in all instances by the term "about." Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

## Claims

1. A compound having the formula: 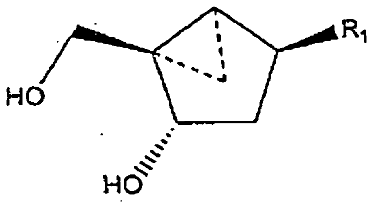 wherein R₁ is a substituted or unsubstituted thymine moiety for use in treating a poxvirus infection in a patient in need thereof.

2. A compound having the formula: 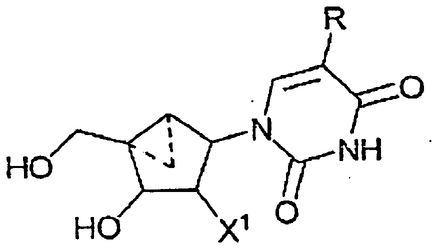 wherein R is -CH₂-X², X¹ is hydrogen; and X² is hydrogen, for use in treating a poxvirus infection in a patient in need thereof.

3. A pharmaceutical composition comprising an effective antiviral amount of a compound having the formula: 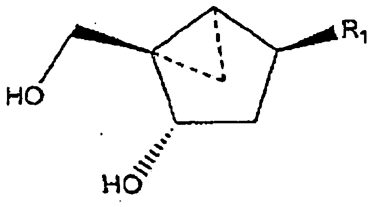 wherein R₁ is a substituted or unsubstituted thymine moiety in a pharmaceuticaly acceptable carrier for use in treating a poxvirus infection in a patient in need thereof.

4. A pharmaceutical composition comprising an effective antiviral amount of a compound having the formula: 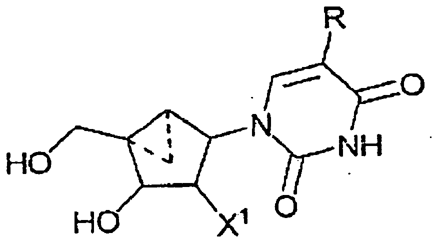 wherein R is -CH₂-X², X¹ is hydrogen; and X² is hydrogen, in a pharmaceutically acceptable carrier for use in treating a poxvirus infection in a patient in need thereof.

5. The compound of claim 2 or the pharmaceutical composition of claim 4, wherein the compound is of the formula: 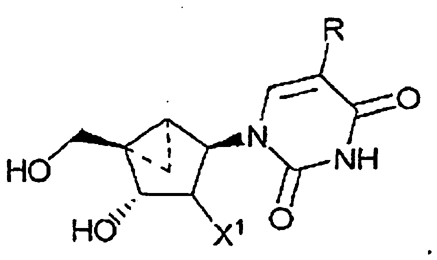 wherein X¹ and R are as defined in claim 2 or in claim 4.

6. A North-methanocarbathymidine triphosphate for use in treating a poxvirus infection in a patient in need thereof.

7. A pharmaceutical composition comprising a North-methanocarbathymidine triphosphate for use in treating a poxvirus infection in a patient in need thereof.

8. Use of a compound having the formula: 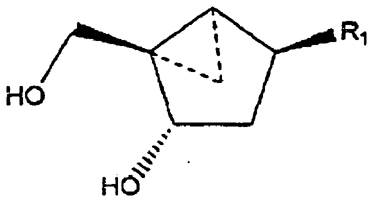 wherein R₁ is a substituted or unsubstituted thymine moiety for the manufacture of a medicament for treating a poxvirus infection in a patient in need thereof.

9. Use of a compound having the formula: 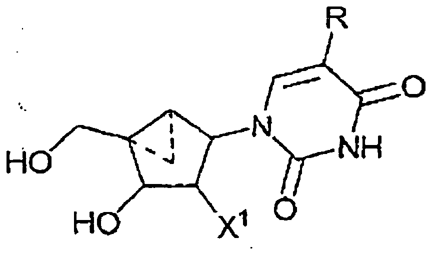 wherein R is -CH₂-X², X¹ is hydrogen; and X² is hydrogen, for the manufacture of a medicament for treating a poxvirus infection in a patient in need thereof.

10. The use of claim 9, wherein the compound is of the formula: 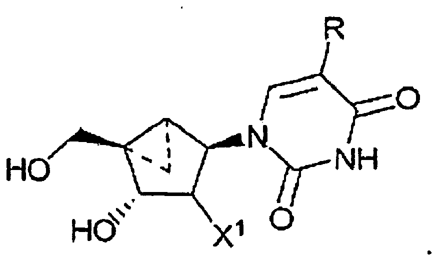 wherein X¹ and R are as defined in claim 9.

11. Use of North-methanocarbathymidine triphosphate for the manufacture of a medicament for treating a poxvirus infection in a patient in need thereof.

12. The compound of claim 2 or 6, the pharmaceutical composition of any one of claims 4 or 7 or the use of any one of claims 9 to 11, wherein the poxvirus is smallpox virus.

13. The compound of claim 2 or 6, the pharmaceutical composition of any one of claims 4 or 7 or the use of any one of claims 9 to 11, wherein the poxvirus is selected from the group consisting of vaccinia, monkeypox, cowpox, rabbitpox, raccoon pox, tatera pox, buffalopox, and camelpox.

14. The compound of claim 2 or 6, the pharmaceutical composition of any one of claims 4 or 7 or the use of any one of claims 9 to 11, wherein the poxvirus is selected from the group consisting of fowl pox, canary pox, goat pox, sheep pox, lumpy skin disease, myxoma, hare fibroma, orf, pseudo-cowpox, swinepox, molluscum contagiosum, tanapox, yaba, and mousepox.

15. The compound of claim 2 or 6, the pharmaceutical composition of claim 4 or 7 or the use of any one of claims 9 to 11 wherein the patient is a human.

16. The use of any one of claims 9 to 11, wherein the medicament is to be administered in an effective antiviral amount.

## Patentansprüche

1. Verbindung mit der Formel: 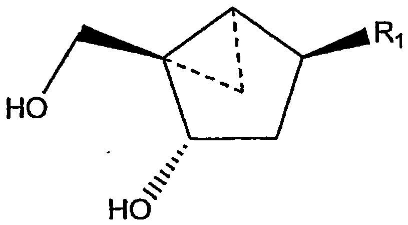 wobei R₁ eine substituierte oder unsubstituierte Thymineinheit ist, zur Verwendung bei der Behandlung einer Pockenvirusinfektion bei einem Patienten, der diese benötigt.

2. Verbindung mit der Formel: 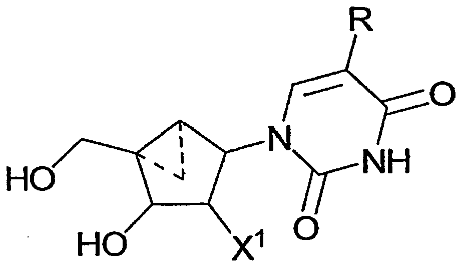 wobei R -CH₂-X² ist, X¹ Wasserstoff ist; und X² Wasserstoff ist, zur Verwendung bei der Behandlung einer Pockenvirusinfektion bei einem Patienten, der diese benötigt.

3. Pharmazeutische Zusammensetzung, umfassend eine antiviral wirksame Menge einer Verbindung mit der Formel: 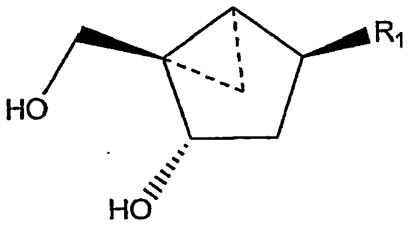 wobei R₁ eine substituierte oder unsubstituierte Thymineinheit ist, in einem pharmazeutisch verträglichen Träger, zur Verwendung bei der Behandlung einer Pockenvirusinfektion bei einem Patienten, der diese benötigt.

4. Pharmazeutische Zusammensetzung, umfassend eine antiviral wirksame Menge einer Verbindung mit der Formel: 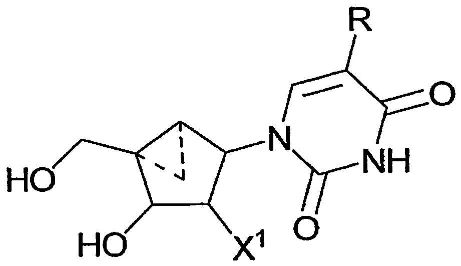 wobei R -CH₂-X² ist, X¹ Wasserstoff ist; und X² Wasserstoff ist, in einem pharmazeutisch verträglichen Träger, zur Verwendung bei der Behandlung einer Pockenvirusinfektion bei einem Patienten, der diese benötigt.

5. Verbindung gemäß Anspruch 2 oder pharmazeutische Zusammensetzung gemäß Anspruch 4, wobei die Verbindung die Formel 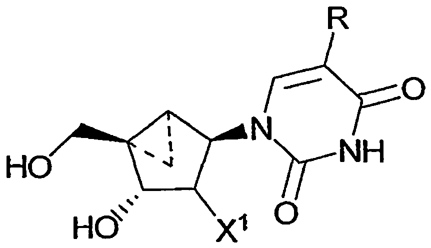 aufweist, wobei X¹ und R wie in Anspruch 2 oder in Anspruch 4 definiert sind.

6. Nord-Methanocarbathymidintriphosphat zur Verwendung bei der Behandlung einer Pockenvirusinfektion bei einem Patienten, der diese benötigt.

7. Pharmazeutische Zusammensetzung, umfassend ein Nord-Methanocarbathymidintriphosphat zur Verwendung bei der Behandlung einer Pockenvirusinfektion bei einem Patienten, der diese benötigt.

8. Verwendung einer Verbindung mit der Formel: 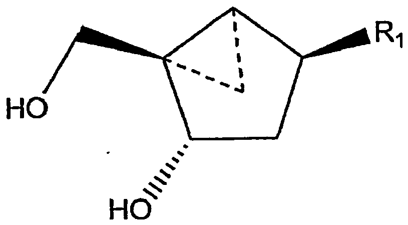 wobei R₁ eine substituierte oder unsubstituierte Thymineinheit ist, zur Herstellung eines Medikaments zur Behandlung einer Pockenvirusinfektion bei einem Patienten, der diese benötigt.

9. Verwendung einer Verbindung mit der Formel: 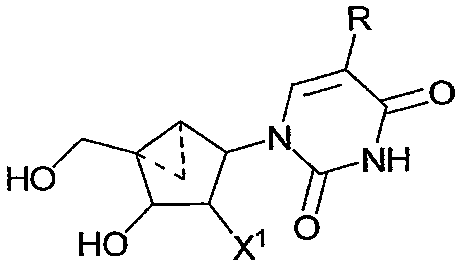 wobei R -CH₂-X² ist, X¹ Wasserstoff ist; und X² Wasserstoff ist, zur Herstellung eines Medikaments zur Behandlung einer Pockenvirusinfektion bei einem Patienten, der diese benötigt.

10. Verwendung gemäß Anspruch 9, wobei die Verbindung die Formel: 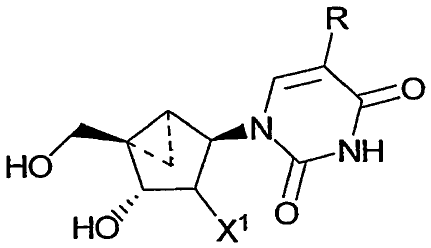 aufweist, wobei X¹ und R wie in Anspruch 9 definiert sind.

11. Verwendung von Nord-Methanocarbathymidintriphosphat zur Herstellung eines Medikaments zur Behandlung einer Pockenvirusinfektion bei einem Patienten, der diese benötigt.

12. Verbindung gemäß Anspruch 2 oder 6, pharmazeutische Zusammensetzung gemäß einem der Ansprüche 4 oder 7 oder Verwendung gemäß einem der Ansprüche 9 bis 11, wobei das Pockenvirus ein Variolavirus ist.

13. Verbindung gemäß Anspruch 2 oder 6, pharmazeutische Zusammensetzung gemäß einem der Ansprüche 4 oder 7 oder Verwendung gemäß einem der Ansprüche 9 bis 11, wobei das Pockenvirus ausgewählt ist aus der Gruppe bestehend aus Vaccinia, Affenpocken, Kuhpocken, Kaninchenpocken, Waschbärpocken, Taterapocken, Büffelpocken und Kamelpocken.

14. Verbindung gemäß Anspruch 2 oder 6, pharmazeutische Zusammensetzung gemäß einem der Ansprüche 4 oder 7 oder Verwendung gemäß einem der Ansprüche 9 bis 11, wobei das Pockenvirus ausgewählt ist aus der Gruppe bestehend aus Geflügelpocken, Kanarienpocken, Ziegenpocken, Schafpocken, Lumpy-skin-Krankheit, Myxoma, Hasenfibrom, Orf, Pseudo-Kuhpocken, Schweinepocken, Molluscum contagiosum, Tanapocken, Yaba-Pocken und Mäusepocken.

15. Verbindung gemäß Anspruch 2 oder 6, pharmazeutische Zusammensetzung gemäß einem der Ansprüche 4 oder 7 oder Verwendung gemäß einem der Ansprüche 9 bis 11, wobei der Patient ein Mensch ist.

16. Verwendung gemäß einem der Ansprüche 9 bis 11, wobei das Medikament in einer antiviral wirksamen Menge zu verabreichen ist.

## Revendications

1. Composé ayant la formule : 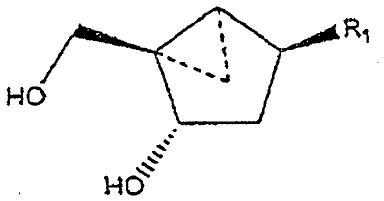 dans laquelle R₁ représente un radical thymine substitué ou non substitué pour une utilisation dans le traitement d'une infection à poxvirus chez un patient en ayant besoin.

2. Composé ayant la formule : 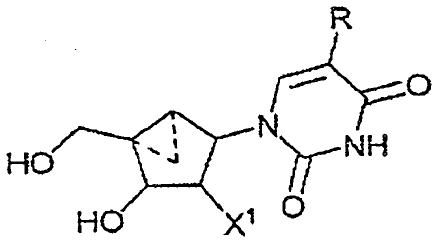 dans laquelle R représente -CH₂-X², X¹ représente un atome d'hydrogène ; et X² représente un atome d'hydrogène, pour une utilisation dans le traitement d'une infection à poxvirus chez un patient en ayant besoin.

3. Composition pharmaceutique comprenant une quantité antivirale efficace d'un composé ayant la formule : 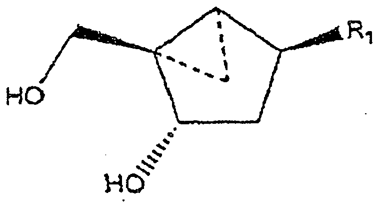 dans laquelle R₁ représente un radical thymine substitué ou non substitué dans un support pharmaceutiquement acceptable pour une utilisation dans le traitement d'une infection à poxvirus chez un patient en ayant besoin.

4. Composition pharmaceutique comprenant une quantité antivirale efficace d'un composé ayant la formule : 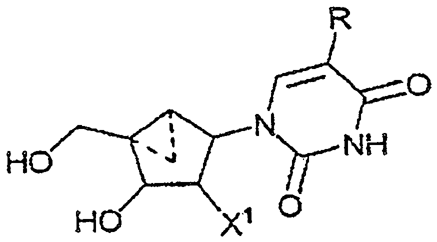 dans laquelle R représente -CH₂-X², X¹ représente un atome d'hydrogène ; et X² représente un atome d'hydrogène, dans un support pharmaceutiquement acceptable pour une utilisation dans le traitement d'une infection à poxvirus chez un patient en ayant besoin.

5. Composé selon la revendication 2 ou composition pharmaceutique selon la revendication 4, ledit composé étant de formule : 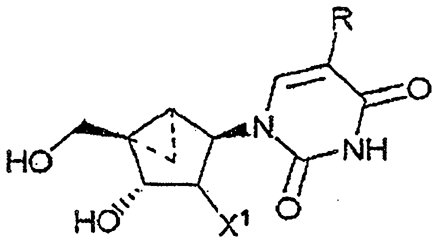 dans laquelle X¹ et R sont tels que définis dans la revendication 2 ou dans la revendication 4.

6. Triphosphate de N-méthanocarbathymidine (North-méthanocarbathymidine) pour une utilisation dans le traitement d'une infection à poxvirus chez un patient en ayant besoin.

7. Composition pharmaceutique comprenant un triphosphate de N-méthanocarbathymidine pour une utilisation dans le traitement d'une infection à poxvirus chez un patient en ayant besoin.

8. Utilisation d'un composé ayant la formule : 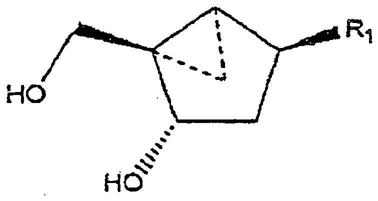 dans laquelle R₁ représente un radical thymine substitué ou non substitué pour la fabrication d'un médicament destiné au traitement d'une infection à poxvirus chez un patient en ayant besoin.

9. Utilisation d'un composé ayant la formule : 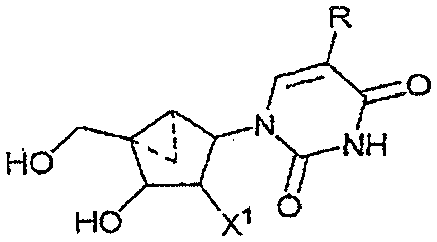 dans laquelle R représente -CH₂-X², X¹ représente un atome d'hydrogène ; et X² représente un atome d'hydrogène, pour la fabrication d'un médicament destiné au traitement d'une infection à poxvirus chez un patient en ayant besoin.

10. Utilisation selon la revendication 9, où le composé est de formule : 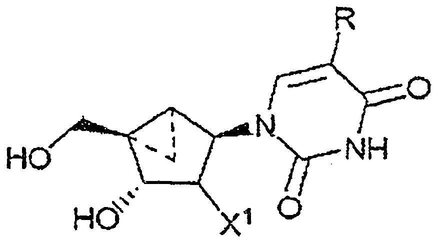 dans laquelle X¹ et R sont tels que définis dans la revendication 9.

11. Utilisation de triphosphate de N-méthanocarbathymidine pour la fabrication d'un médicament destiné au traitement d'une infection à poxvirus chez un patient en ayant besoin.

12. Composé selon la revendication 2 ou 6, composition pharmaceutique selon l'une quelconque des revendications 4 ou 7 ou utilisation selon l'une quelconque des revendications 9 à 11, où le poxvirus est le virus de la variole.

13. Composé selon la revendication 2 ou 6, composition pharmaceutique selon l'une quelconque des revendications 4 ou 7 ou utilisation selon l'une quelconque des revendications 9 à 11, où le poxvirus est choisi dans le groupe constitué par la vaccine, la variole du singe, la variole bovine, la variole du lapin, la variole du raton laveur, la variole de la gerbille, la variole du buffle et la variole du chameau.

14. Composé selon la revendication 2 ou 6, composition pharmaceutique selon l'une quelconque des revendications 4 ou 7 ou utilisation selon l'une quelconque des revendications 9 à 11, où le poxvirus est choisi dans le groupe constitué de la variole aviaire, la variole du canari, la variole de la chèvre, la variole du mouton, la dermatose nodulaire, le myxome, le fibrome du lièvre, l'ecthyma contagieux, la pseudo-variole bovine, la variole porcine, le molluscum contagiosum, le tanapox, le yaba et la variole du rongeur.

15. Composé selon la revendication 2 ou 6, composition pharmaceutique selon la revendication 4 ou 7 ou utilisation selon l'une quelconque des revendications 9 à 11, où le patient est un être humain.

16. Utilisation selon l'une quelconque des revendications 9 à 11, où le médicament doit être administré dans une quantité antivirale efficace.


### SEQUENCE LISTING

<110> The Government of the United States of America, as represented by the Secretary, Department of Health and Human Services;
   Tseng, Christopher K.;
   Marquez, Victor E.
<120> NORTH-2'-DEOXY-METHANOCARBATHYMIDINES AS
   ANTIVIRAL AGENTS AGAINST POXVIRUSES
<130> NIH326.001VPC
<150> US 60/684,811
   <151> 2005-05-25
<160> 1
<170> FastSEQ for Windows Version 4.0
<210> 1
   <211> 15
   <212> DNA
   <213> Artificial Sequence
<220>
   <223> synthetic oligonucleotide
<400> 1
   cttcattttt tcttc 15